# EUROPÄISCHE PATENTSCHRIFT

(11) **EP 2 655 361 B1**
(45) Veröffentlichungstag und Bekanntmachung des Hinweises auf die Patenterteilung: **24.02.2016**
(21) Anmeldenummer: 11802378.7
(22) Anmeldetag: 19.12.2011
(51) Int. Cl.: C07D 403/04

(54) **VERFAHREN ZUR HERSTELLUNG VON TRIAZINYLSUBSTITUIERTEN OXINDOLEN**
PROCESS FOR THE PREPARATION OF TRIAZINYL SUBSTITUTED OXINDOLES
PROCÉDÉ DE PRÉPARATION D'OXINDOLES SUBSTITUÉS PAR TRIAZINYLE

(30) Priorität: 21.12.2010 EP 10196205; 21.12.2010 US 201061425349 P
(43) Veröffentlichungstag der Anmeldung: 30.10.2013
(73) Patentinhaber: Bayer Intellectual Property GmbH, 40789 Monheim am Rhein (DE)
(72) Erfinder: KARIG, Gunter, 65719 Hofheim am Taunus (DE); FORD, Mark, James, 61389 Schmitten (DE); SIEGEL, Konrad, 40597 Düsseldorf (DE)
(74) Vertreter: BIP Patents
(86) Internationale Anmeldenummer: PCT/EP2011/073283
(87) Internationale Veröffentlichungsnummer: WO 2012/084855

(56) Entgegenhaltungen:
- WO-A2-2006/136606
- JP-A- 2000 044 546
- US-A1- 2004 116 388
- CHENG-KANG MAI ET AL: "alpha-Arylation of 3-Aryloxindoles", ORGANIC LETTERS, AMERICAN CHEMICAL SOCIETY, US, Bd. 12, Nr. 10, 21. Mai 2010 (2010-05-21), Seiten 2306-2309, XP008138081, ISSN: 1523-7060, DOI: DOI:10.1021/OL100666V [gefunden am 2010-04-28] in der Anmeldung erwähnt

## Beschreibung

Die vorliegende Anmeldung betrifft verbesserte Verfahren zur Herstellung von triazinylsubstituierten Oxindolen und deren Verwendung als Zwischenprodukte für die Synthese von Feinchemikalien und von Wirkstoffen im Bereich der Landwirtschaft.

In 3-Position substituierte Oxindole stellen ein wichtiges Strukturmotiv bei einer Reihe von Naturstoffen und pharmazeutisch wirksamen Substanzen dar. Einige dieser Verbindungen zeigen biologische Aktivität gegen verschiedene Krankheitsbilder und haben z.B. Antitumor- oder Anti-HIV-Eigenschaften (Ding et al., J. Med. Chem. 2006, 49, 3432; Jiang et al., Bioorg. Med. Chem. Lett. 2006, 16, 2105).

Eine weitere Untergruppe der in 3-Position heteroaromatisch substituierten Oxindole sind die 3-Triazinyloxindole (3-(1,3,5-Triazin-2-yl)-1,3-dihydro-2H-indol-2-one. Die Herstellung dieser, mit der Trivialbezeichnung als "3-Triazinyloxindole" bezeichneten Verbindungen, ist Gegenstand der vorliegenden Erfindung.

Es ist bekannt, dass ein an ein aromatisches, heteroaromatisches oder an ein aliphatisches Kohlenstoffgerüst gebundener Wasserstoff gegen funktionelle Substituenten, die ebenfalls aromatisch, heteroaromatisch oder aliphatisch sein können, ausgetauscht werden kann.

Interessant ist dabei, dass die Reaktionsbedingungen für die Substitution des Wasserstoffs in 3-Position von Oxindolen, abhängig von der Natur der Substituenten, verschieden sind. Entsprechend wurden die Reaktionsbedingungen für den Austausch gegen aliphatische, aromatische und heteroaromatische Reste unabhängig voneinander erforscht und entwickelt.

Zu den Standardreaktionen der Substitution von Oxindolen in 3-Position gehört der Austausch von Wasserstoff gegen aliphatische Substituenten (Science of Synthesis, 10 (2000), p.600).

Der Austausch gegen aromatische Substituenten in Gegenwart von Palladium wurde von Taylor et al. (J. Am. Chem. Soc., 2009, 131, 9900-9901), sowie von Altman et al. (J. Am. Chem. Soc. 2008, 130 (29), 9613 - 9620) sowie von Durbin et al. (Org. Lett., 2008, 10 (7), 1413-1415) beschrieben.

Die Synthese von substituierten Oxindolen, bei welchen der Wasserstoff gegen heteroaromatische 6-Ring-Substituenten ausgetauscht wurde, ist ebenfalls beschrieben. Beispielhaft genannt werden hier die Substitution in 3-Position von N-Methyloxindol mit einem substituierten Pyridazin (Shen et al., Org. Lett., 2006, 8, 1447 - 1450), die Herstellung von substituierten 3-(Chinazolin-4-yl)-oxindolen (US 6265411), die Substitution in 3-Position von Oxindol mit substituierten Chinazolinen an einer festen Phase (Hennequin et al., Tetrahedron Lett., 1999 , 40, 3881 - 3884), die Substitution mit substituierten Pyridinen oder Pyridin-N-oxiden (z. B.

US 2009/291982, WO 2007/89193, WO 2005/27823, WO 2003/82853), die Herstellung von substituierten 3-(Pyrimidin-4-yl)-oxindolen (WO 2006/136606, WO 2003/82853, US 2007/281949) oder die Herstellung von substituierten 3-(2H-Pyrazolo[3,4-d]pyrimidin-4-yl)-oxindolen (US 2007/281949).

Gemäß Schema 1 können 3-Triazinyloxindole durch Austausch eines Wasserstoffatoms in 3-Position eines wahlweise substituierten Oxindols (1) mit einem wahlweise substituierten Triazin (2), welches eine geeignete Abgangsgruppe X trägt, in Gegenwart einer "geeigneten" Base erhalten werden.

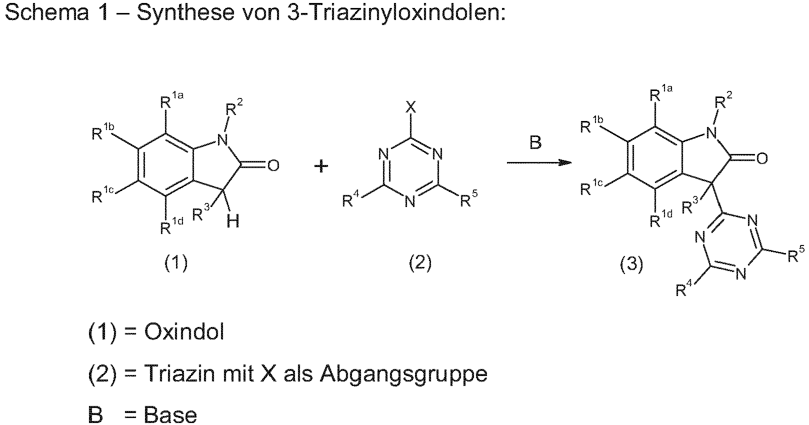

Dabei ist bekannt, dass der für den Austausch des Wasserstoffs wichtige Schritt der Deprotonierung des Oxindols, durch die Wahl des Substituenten R³ gezielt beeinflusst werden kann.

Den im vorgenannten Stand der Technik offenbarten Reaktionen ist gemeinsam, dass das eingesetzte Oxindol zuerst mit einer starken Base deprotoniert wird und anschließend die heterocyclische Komponente, typischerweise als Chlor-Verbindung, zugegeben wird.

Zur Deprotonierung werden im Stand der Technik starke, wasserempfindliche Basen, wie Natriumhexamethyldilsilazan oder Lithiumdiisopropylamid (LDA), Natriumhydrid oder Lithiumhydrid, eingesetzt.

Nachteiligerweise führt der Einsatz der Basen Natriumhydrid und Lithiumhydrid zur Entstehung von äquimolaren Mengen elementaren Wasserstoffs. Außerdem müssen die im Zusammenhang mit diesen Basen eingesetzten Lösungsmittel vor ihrem Einsatz aufwendig getrocknet werden.

Eine analoge Kupplung - analog zu der in WO 2005/061519 beschriebenen Kupplung von Oxindolen mit Chinolinen, bei der Chinolin-N-Oxide in Gegenwart von Essigsäureanhydrid eingesetzt werden - mit Triazinen der Formel (2) ist nicht bekannt.

In Schema 2 zusammengefasst ist ein bekanntes Verfahren zur Herstellung von substituierten 3-Triazinyloxindolen. Diese sind dadurch gekennzeichnet, dass sie am Triazinring Stickstoff-Substituent tragen. Die Synthese ist in US 2004/116388, WO 2002/083654 und WO 2001/025220 offenbart.

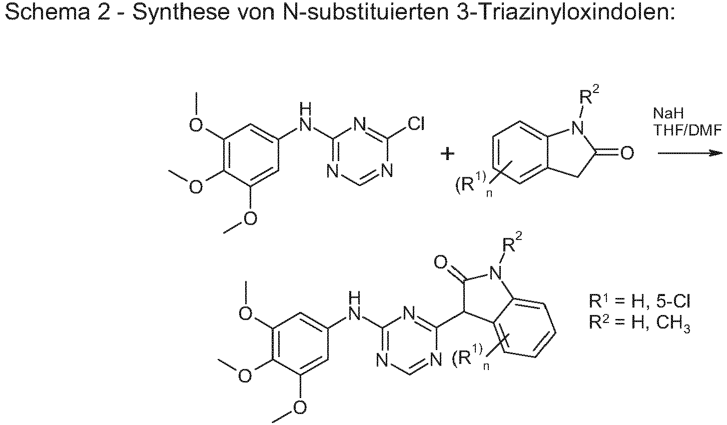

Als triazinhaltige Komponente wurde in der Reaktion gemäß Schema 2 ein substituiertes 4-Chlor-N-phenyl-1,3,5-triazin-2-amin verwendet. Die Reaktion erfolgte durch Deprotonierung des eingesetzten Oxindols in DMF/THF mit Natriumhydrid, gefolgt von der Zugabe der Triazinkomponente und anschließendem Rühren der Reaktionsmischung bei 80°C.

Nachteiligerweise liegen die erzielten Ausbeuten bei dieser bekannten Synthese nur bei 2.5%, bzw. 7% für die am Stickstoff unsubstituierten Oxindole (R² = H) und bei 29% für das N-Methyloxindol (R² = Me).

Die Nachteile des beschriebenen Verfahrens betreffen, neben den sehr niedrigen Ausbeuten, auch die Verwendung von starken Basen wie Natriumhydrid, die zur Entstehung von äquimolaren Mengen elementaren Wasserstoffs führen, welche technisch schwer handhabbar sind. Daher ist das beschriebene Verfahren keine gangbare Lösung für den technischen (industriellen) Maßstab.

In dem in US 2004/116388 für die Verbindung mit der Nummer 380 beschriebenen Verfahren zur Herstellung von substituierten 3-Triazinyloxindolen werden auf ein Äquivalent der Oxindol-Komponente nur 0,4 Äquivalente der Triazinkomponente eingesetzt. Bezogen auf die Oxindol-Komponente kann dies lediglich zu einer maximalen theoretischen Ausbeute von 40 % führen. Eine Erhöhung der Ausbeute kann durch den Einsatz eines Überschusses an Oxindol erreicht werden. Da die Oxindol-Komponente, abhängig vom Substitutionsmuster, das eher wertvollere Edukt sein kann, ist diese Reaktionsführung unter Verwendung eines 2.5-fachen Überschusses an Oxindol im technischen Maßstab als nachteilig anzusehen.

Es wurde bereits darauf hingewiesen, dass die Reaktionsbedingungen für den Austausch des Wasserstoff in 3-Position von Oxindolen gegen aliphatische, aromatische und heteroaromatische Substituenten jeweils unabhängig voneinander etabliert werden mussten, weil die Art der einzuführenden Substituenten die Reaktion stark beeinflussen kann.

Das Gleiche scheint wiederum für die weitere Verzweigung, d.h. die weitere Substitution dieser Substituenten, insbesondere für die weitere Substitution der heteroaromatischen Substituenten, zu gelten.

So ist im Stand der Technik keine technisch geeignete Synthese von 3-Triazinyloxindolen, welche am Triazinring Alkyl- oder Alkoxysubstituenten tragen, beschrieben.

Die Anwendung der bislang bekannten Herstellungsverfahren in der Synthese von 3-Triazinyloxindolen, welche am Triazinring Alkyl- oder Alkoxysubstituenten tragen, liefert im technischen (industriellen) Maßstab keine befriedigenden Ergebnisse.

Zu Vergleichszwecken wurden die im Dokument US 2004/116388 beschriebenen Bedingungen in der Umsetzung von 7-Fluor-1,3-dihydro-2H-indol-2-on (Beispiel 1 Variante F), bzw. 1,3-Dihydro-2H-indol-2-on (Beispiel 2 Variante B) mit 2-Chlor-4,6-dimethoxy-1,3,5-triazin angewendet.

Dabei zeigte sich, dass die erzielten Ausbeuten, jeweils bezogen auf die Oxindol-Komponente nur bei 39% (Beispiel 1 Variante F), bzw. nur bei 34% (Beispiel 2 Variante B) liegen. Werden diese Bedingungen bei der Umsetzung der Edukte in einem technisch vorteilhaften Verhältnis, nämlich 1 Äquivalent der Oxindol-Komponente mit 1.2 Äquivalenten der Triazinkomponente, angewandt, dann liegen die erzielten Ausbeuten bei 39% (Beispiel 1 Variante G) bzw. 30% (Beispiel 2 Variante C).

In Organic Letters (2010) 2306-2309 wird in den in Tabelle 4 beschriebenen Arylierungsreaktionen als Edukt jeweils 3-Phenyloxindol eingesetzt, also ein Oxindol, welches in 3-Position einen Phenylsubstituenten trägt. Dieses 3-Phenyloxindol wird mit elektronenarmen Chlorbenzolderivaten und 5-Halooxazolen in Gegenwart von Cäsiumcarbonat in 3-Position aryliert.

Bekanntermaßen wird die Acidität von Methylgruppen oder Methylengruppen durch Austausch eines Wasserstoffsubstituenten gegen einen Phenylsubstituenten in der Regel stark erhöht. Dies führt zu einem um mehrere Größenordnungen erniedrigten pKa-Wert des oder der verbliebenen Wasserstoffsubstituenten an Methylgruppe oder Methylengruppe.

In einer Reihe von Publikationen finden sich entsprechende Beispiele, in denen pKa-Werte von organischen oder anorganischen Verbindungen in Wasser oder in organischen Lösemitteln wie Dimethylsulfoxid beschrieben werden. Die pKa-Werte in organischen Lösemitteln wurden entweder direkt gemessen oder über andere Methoden extrapoliert. So ist in Acc. Chem. Res. 1988, 21, 456 in Tabelle II für 4-Methylpyridin ein pKa-Wert von 35 (extrapoliert für DMSO) und für 4-Benzylpyridin ein pKa-Wert von 26.7 (in DMSO) angegeben. Ebenfalls in Acc. Chem. Res. 1988, 21, 456 in Tabelle 11 ist für (Methylsulfanyl)benzol ein pKa-Wert von 42 (extrapoliert für DMSO), für (Benzylsulfanyl)benzol ein pKa-Wert von 30.8 (in DMSO) und für Diphenylmethyl-phenylsulfid ein pKa-Wert von 26.8 (in DMSO) angegeben. Für Oxindol ist in Acc. Chem. Res. 1988, 21, 456 in Tabelle II ein pKa-Wert von 18.2 (in DMSO) angegeben.

An den genannten Beispielen wird die Erhöhung der Acidität von Methylgruppen oder Methylengruppen um mehrere Größenordnungen durch Austausch eines Wasserstoffsubstituenten gegen einen Phenylsubstituenten sehr deutlich.

In den Beispielen der vorliegenden Anmeldung wurden als Edukte nur Oxindole eingesetzt, welche in 3-Position zwei Wasserstoffatome tragen. In Organic Letters (2010) 2306-2309 werden dagegen 3-Phenyloxindole als Edukte eingesetzt. Die Edukte unterscheiden sich somit in ihrer Acidität. Oxindole, welche in 3-Position zwei Wasserstoffatome tragen, sind weniger acide als das in Organic Letters (2010) 2306-2309 in den Reaktionen der Tabelle 4 eingesetzte 3-Phenyloxindol.

Es überrascht daher nicht, dass in den Literaturbeispielen, in denen Arylierungsreaktionen an in 3-Position unsubstituierten Oxindolen beschrieben werden, starke Basen wie Natriumhydrid verwendet werden. Dass auch für Oxindole, die in 3-Position einen Substituenten tragen, welcher die Acidität in nichtwässrigen Lösungsmitteln um mehrere Größenordnungen verringert, zur Deprotonierung schwächere Basen wie Cäsiumcarbonat verwendet werden können war für den Fachmann zu erwarten und wurde in Organic Letters (2010) 2306-2309 bestätigt.

Jedoch ist für den Fachmann überraschend, dass entsprechend der Lehre der Erfindung eine Arylierungsreaktionen an in 3-Position unsubstituierten Oxindolen (Oxindole die in 3-Position zwei Wasserstoffe aufweisen) wider Erwarten auch mit schwächeren Basen wie Kaliumcarbonat oder Natriumhydroxid in guten Ausbeuten möglich ist.

Um die Anwendbarkeit der in Organic Letters (2010) 2306 (Supplement Seite S-12 General Procedure) beschriebenen Bedingungen zur Arylierung von 3-Aryloxindolen mit elektronenarmen Chlorbenzolderivaten und 5-Halooxazolen (Tabelle 4 in Organic Letters (2010) 2306) auch bei Verwendung von Chlortriazinen zu prüfen, wurde 3-Phenyl-1,3-dihydro-2H-indol-2-on mit 2-Chlor-4,6-dimethoxy-1,3,5-triazin in Gegenwart von Cäsiumcarbonat in N,N-Dimethylformamid umgesetzt (siehe Beispiel 10). Da die Reaktion sehr schnell auch bei Raumtemperatur ablief, wurde auf eine erhöhte Temperatur und eine verlängerte Reaktionszeit verzichtet. Das in der Reaktionsmischung enthaltene Produkt wurde durch Säulenchromatographie gereinigt. Eine Strukturaufklärung mittels 2D-NMR belegt, dass es sich bei dem erhaltenen Produkt allerdings nicht um das gewünschte in 3-Position arylierte Produkt (3-(4,6-Dimethoxy-1,3,5-triazin-2-yl)-3-phenyl-1,3-dihydro-2H-indol-2-on), sondern um das O-arylierte Produkt (2-[(4,6-Dimethoxy-1,3,5-triazin-2-yl)oxy]-3-phenyl-1H-indol) handelt.

In der Folge wurden, wiederum zu Vergleichszwecken, die in Organic Letters (2010) 2306 (Supplement Seite S-12 General Procedure) beschriebenen Bedingungen zur Arylierung ebenfalls unter Verwendung von Chlortriazinen angewendet, und zwar zur Arylierung von einem in 3-Position unsubstituierten Oxindol. Dazu wurde 7-Fluor-1,3-dihydro-2H-indol-2-on mit 2-Chlor-4,6-dimethoxy-1,3,5-triazin in Gegenwart von Cäsiumcarbonat in N,N-Dimethylformamid umgesetzt (Beispiel 1 Variante H). Jedoch wurde als Reaktionsprodukt die in 3-Position arylierte Titelverbindung mit nur 22% Ausbeute erhalten. Als Hauptprodukte wurden im isolierten Feststoff sowie in der eingeengten Mutterlauge mehrfach arylierte Produkte erhalten. Auch konnte durch HPLC-Analytik gezeigt werden, dass das als Edukt eingesetzte Oxindol nicht vollständig abreagiert hatte.

Somit ist das in Organic Letters (2010) 2306 (Supplement Seite S-12 General Procedure) beschriebene Verfahren nicht geeignet, 3-Triazinyloxindole im technischen Maßstab herzustellen, zumindest dann, wenn 2-Chlor-4,6-dimethoxy-1,3,5-triazin als Arylierungsreagenz verwendet wird.

Die Aufgabe der Erfindung besteht vor diesem Hintergrund in der Bereitstellung eines verbesserten Verfahrens, das im technischen Maßstab eine im Vergleich zu den bekannten Verfahren vereinfachte Herstellung von 3-Triazinyloxindolen, bei zugleich verbesserter Gesamtausbeute, ermöglicht.

Überraschend wurde nun gefunden, dass sich die schwächeren Basen Kaliumcarbonat oder Natriumcarbonat sowie Lithiumhydroxid, Natriumhydroxid, Kaliumhydroxid, Bariumhydroxid oder Tertalkylammoniumhydroxid, sowie Kaliumphosphat (K₃PO₄), K₂HPO₄ oder Natriumphosphat oder Mischungen bestehend aus mindestens zwei der vorgenannten Basen zur Lösung der Aufgabe eignen.

Die genannten Basen haben den Vorteil, dass sie für den Einsatz im technischen Maßstab geeignet sind, da sie insbesondere in Gegenwart von Wasser nicht zersetzlich sind sowie keine äquimolaren Mengen Wasserstoff erzeugen und zugleich zu einer deutlich verbesserten Gesamtausbeute führen.

Somit wird die Aufgabe gelöst durch ein Verfahren zur Herstellung von Verbindungen der Formel (3) 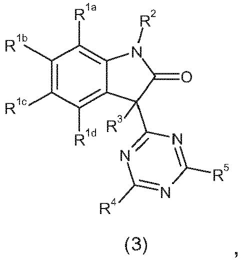 worin
R^{1a} bis R^{1d} unabhängig voneinander ausgewählt sind aus der Gruppe bestehend aus Wasserstoff, Fluor, Chlor, Brom, lod sowie aus
(C₁-C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₈)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₁-C₆)-Alkylthio, wobei der Alkylthiorest verzweigt oder unverzweigt ist und unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der Fluor, Chlor, (C₁-C₄)-Alkyl oder (C₁-C₄)-Alkoxy bestehenden Gruppe, substituiert ist, und Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, lod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R² für
Wasserstoff,
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist, oder
Benzyl, wobei das Benzyl unsubstituiert ist oder substituiert ist durch einen oder mehrere Substituenten, ausgewählt aus der Gruppe bestehend aus Fluor, Chlor, Brom, lod, Nitro, (C₁-C₄)-Alkyl, (C₁-C₄)-Alkoxy oder (C₃-C₇)-Cycloalkyl oder (C₁-C₄)-Alkylthio sowie aus COOR^{a}, worin R^{a} für ein (C₁-C₄)-Alkyl steht, und -CONR^{b'}R^{b"} oder -CONHR^{b"}worin R^{b'} und R^{b"} jeweils unabhängig voneinander für ein (C₁-C₄)-Alkyl steht, wobei jeweils zwei Substituenten am N-Atom zusammen gegebenenfalls einen unsubstituierten oder substituierten Ring bilden,
steht,
R³ für Wasserstoff oder Methyl,
steht,
R⁴ und R⁵ unabhängig voneinander jeweils für
Wasserstoff,
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
stehen,
wobei ein Oxindol (1) 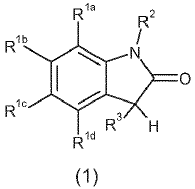 worin
R^{1a} bis R^{1d} sowie R² und R³ wie in Formel (3) definiert sind, in einem Lösungsmittel umgesetzt wird mit einem Triazin (2) 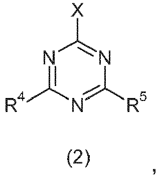 worin
R⁴ und R⁵ wie in Formel (3) definiert sind, und
X als Abgangsgruppe für Cl, Br, I, Alkoxy, Alkylsulfonyl, (Alkylsulfonyl)oxy, Haloalkylsulfonyl, Phenylsulfonyl oder Toluol-4-Sulfonyl steht, dadurch gekennzeichnet, dass die Umsetzung in Gegenwart von
- Kaliumcarbonat oder Natriumcarbonat,
- Lithiumhydroxid, Natriumhydroxid, Kaliumhydroxid, Bariumhydroxid oder Tertalkylammoniumhydroxid,
- Kaliumphosphat (K₃PO₄), Kaliumhydrogenphosphate (K₂HPO₄) oder Natriumphosphat, oder
- in einer mindestens zwei der vorgenannten Basen umfassenden Mischung erfolgt.

Soweit in dieser Anmeldung auf ein "Oxindol" verwiesen wird, ist eine der von der allgemeinen Formel (1) umfassten Verbindungen gemeint. Die als Edukte eingesetzten Oxindole (1) 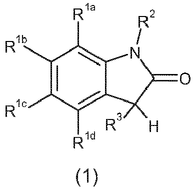 worin die Reste R^{1a} bis R^{1d}, R² und R³ wie oben definiert sind, sind bekannt oder können unter Anwendung der dem Fachmann bekannten Verfahren hergestellt werden. Soweit in dieser Anmeldung auf ein "Triazin" oder eine "Triazinkomponente" verwiesen wird, ist eine der von der allgemeinen Formel (2) umfassten Verbindungen gemeint. Die ebenfalls als Edukte eingesetzten Triazine (2) 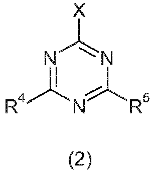 worin R⁴, R⁵ und X wie oben definiert sind, sind ebenfalls bekannt oder können unter Anwendung von dem Fachmann bekannten Verfahren hergestellt werden.

Im Hinblick auf die erfindungsgemäßen Verbindungen werden die vorstehend und weiter unten verwendeten Bezeichnungen zusammenfassend erläutert. Diese sind dem Fachmann geläufig und haben insbesondere die im Folgenden erläuterten Bedeutungen:

Die Bezeichnung "Halogen" bedeutet beispielsweise Fluor, Chlor, Brom oder lod. Wird die Bezeichnung für einen Rest verwendet, dann bedeutet "Halogen" beispielsweise ein Fluor-, Chlor-, Brom- oder Iodatom.

Alkyl bedeutet einen geradkettigen oder verzweigten offenkettigen, gesättigten Kohlenwasserstoffrest.

Der Ausdruck "(C₁-C₄)Alkyl" bedeutet eine Kurzschreibweise für Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)Alkyl", umfassen entsprechend auch gradkettige oder verzweigte Alkylreste mit einer größeren Zahl von C-Atomen, d. h. gemäß Beispiel auch die Alkylreste mit 5 und 6 C-Atomen.

Cycloalkyl bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl.

Im Falle von substituiertem Cycloalkyl werden auch spirocyclische aliphatische Systeme umfasst, wie beispielsweise Spiro[2.2]pent-1-yl, Spiro[2.3]hex-1-yl, Spiro[2.3]hex-4-yl, 3-Spiro[2.3]hex-5-yl.

Aryl bedeutet ein mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Vom Begriff "gegebenenfalls substituierte Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist.

Von der Systematik her ist Aryl in der Regel auch von dem Begriff "gegebenenfalls substituiertes Phenyl" umfasst.

Alkoxy bedeutet ein über ein Sauerstoffatom gebundenen Alkylrest, Alkenyloxy bedeutet ein über ein Sauerstoffatom gebundenen Alkenylrest, Alkinyloxy bedeutet ein über ein Sauerstoffatom gebundenen Alkinylrest, Cycloalkyloxy bedeutet ein über ein Sauerstoffatom gebundenen Cycloalkylrest und Cycloalkenyloxy bedeutet ein über ein Sauerstoffatom gebundenen Cycloalkenylrest.
Alkylthio bedeutet ein über ein Schwefelatom gebundenen Alkylrest, Alkenylthio bedeutet ein über ein Schwefelatom gebundenen Alkenylrest, Alkinylthio bedeutet ein über ein Schwefelatom gebundenen Alkinylrest, Cycloalkylthio bedeutet ein über ein Schwefelatom gebundenen Cycloalkylrest und Cycloalkenylthio bedeutet ein über ein Schwefelatom gebundenen Cycloalkenylrest.

Haloalkyl, -alkenyl und -alkinyl bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie CH₂CH₂Cl, CH₂CH₂F, CHClCH₃, CHFCH₃, CH₂Cl, CH₂F; Perhaloalkyl wie CCl₃ oder CF₃ oder CF₂F₃; Polyhaloalkyl wie CHF₂, CH₂F, CH₂CHFCl, CHCl₂, CF₂CF₂H, CH₂CF₃,; Haloalkoxy ist z.B. OCF₃, OCHF₂, OCH₂F, OCF₂CF₃, OCH₂CF₃ und OCH₂CH₂Cl; Entsprechendes gilt für Haloalkenyl und andere durch Halogen substituierten Reste.

Mit der Definition "mit einem oder mehreren Resten substituiert ist" sind, wenn nicht anders definiert, unabhängig voneinander ein oder mehrere gleiche oder verschiedene Reste gemeint, wobei zwei oder mehrere Reste an einem Cyclus als Grundkörper einen oder mehrere Ringe bilden können.

Die jeweils unsubstitutierten oder substituierten Reste können verzweigt und unverzweigt sein. So umfasst zum Beispiel ein mit "C₄-Alkyl" bezeichneter Rest neben dem unverzweigten Butyl-Rest alle weiteren C₄-Isomere darunter auch *tert-*Butyl.

Wenn zwei oder mehrere Reste einen oder mehrere Ringe bilden, so können diese carbocyclisch, heterocyclisch, gesättigt, teilgesättigt, ungesättigt, beispielsweise auch aromatisch und gegebenenfalls weiter substituiert sein. Die annellierten Ringe sind vorzugsweise 5- oder 6-Ringe, besonders bevorzugt sind benzokondensierte Cyclen.

Der Kern des erfindungsgemäßen Verfahrens besteht in der Umsetzung der Edukte der Formel (1) und (2) in der Gegenwart von Basen, welche sich dadurch auszeichnen, dass sie sich in Gegenwart von Wasser nicht zersetzen und außerdem während der Reaktion keinen Wasserstoff (H₂) freisetzen.

Einige sehr starke bis mittelstarke Basen, wie z.B. Natriumhydrid (NaH), reagieren in Wasser unter Zersetzung und sind daher für die technische Anwendung nicht geeignet. Basen mit diesen Nachteilen sind nur im Labormaßstab sicher handhabbar. Für die technische, d.h. industrielle, Anwendung sind starke Basen, wie NaH, daher nicht geeignet.

Bei der erfindungsgemäßen Umsetzung als Base eingesetzt werden
- Kaliumcarbonat oder Natriumcarbonat, sowie
- Lithiumhydroxid, Natriumhydroxid, Kaliumhydroxid, Bariumhydroxid oder Tertalkylammoniumhydroxid, sowie
- Kaliumphosphat (K₃PO₄), K₂HPO₄ oder Natriumphosphat, oder
- Mischungen aus mindestens zwei der vorgenannten Basen.

Die genannten Carbonate, Hydroxide und Phosphate haben gegenüber starken Basen, wie z.B. Natriumhydrid, den wesentlichen Vorteil, dass sie technisch (industriell) geeigneter sind, da bei ihrer Verwendung keine äquimolaren Mengen an Wasserstoff entstehen und die genannten schwächeren Basen sich auch nicht in Gegenwart von Wasser zersetzen.

Besonders bevorzugte Basen sind Kaliumcarbonat, Natriumcarbonat, Natriumhydroxid, Kaliumhydroxid oder eine mindestens zwei-komponentige Mischung bestehend aus mindestens einem der beiden Carbonate: Kaliumcarbonat und Natriumcarbonat sowie aus mindestens einem der beiden Hydroxide: Kaliumhydroxid oder Natriumhydroxid.

Die vier besonders bevorzugten zwei-komponentigen Mischungen betreffen somit die Mischungen bestehend aus Kaliumcarbonat und Kaliumhydroxid, Kaliumcarbonat und Natriumhydroxid, Natriumcarbonat und Kaliumhydroxid sowie die Mischung bestehend aus Kaliumcarbonat und Natriumhydroxid.

Daneben sind natürlich weitere Mischungen denkbar, die jeweils mehr als zwei der oben als für die Umsetzung besonders bevorzugt genannten Basen enthalten.

Da die erfindungsgemäß eingesetzten Basen Wasser enthalten, bzw. erzeugen oder freisetzen können, Triazine (2) in Gegenwart von Wasser jedoch leicht hydrolysieren, muss es als überraschend angesehen werden, dass die erfindungsgemäß eingesetzten Basen sich überhaupt als zur Lösung der Aufgabe geeignet erweisen konnten.

In einer bevorzugten Ausführungsform des erfindungsgemäßen Verfahrens sind die Reste R^{1a} bis R^{1d} in den Verbindungen der Formeln (3) und (1) unabhängig voneinander ausgewählt aus der Gruppe bestehend aus Wasserstoff, Fluor, Chlor, Brom, lod sowie aus
(C₁-C₆)-Alkyl, wobei der Alkylrest verzweigt oder unverzweigt ist und unsubstituiert ist oder durch einen oder mehrere Substituenten, ausgewählt aus der Gruppe bestehend aus Fluor und Chlor substituiert ist, und
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist.

In einer besonders bevorzugten Ausführungsform sind die Reste R^{1a} bis R^{1d} in den Verbindungen der Formeln (3) und (1) unabhängig voneinander ausgewählt aus der Gruppe bestehend aus Fluor, Chlor und Trifluormethyl (CF₃), Trifluormethoxy (O-CF₃) und Methoxy (O-Me).

In einer ganz besonders bevorzugten Ausführungsform steht der Rest R^{1a} für Fluor oder Chlor, d.h. die Verbindungen der Formeln (3) und (1) sind in 7-Position durch Fluor (7-Fluor) oder Chlor (7-Chlor) substituiert.

Ebenfalls ganz besonders bevorzugt sind Verbindungen der Formeln (3) und (1), welche in 5-Position durch Fluor (5-Fluor) substituiert sind, d.h. der Rest R^{1c} steht für Fluor (siehe Beispiel 5).

Ganz besonders bevorzugt sind weiterhin auch Verbindungen der Formeln (3) und (1), welche in 7-Position durch Fluor (7-Fluor) und zugleich in 5-Position durch Fluor (5-Fluor) substituiert sind, d.h. die Reste R^{1a} und R^{1c} stehen für Fluor (siehe Beispiel 3).

In einer weiteren besonders bevorzugten Ausführungsform steht der Rest R² in den Formeln (3) und (1) für
- Wasserstoff, oder für jeweils
- unsubstituiertes Methyl, Ethyl und Benzyl.

In einer ganz besonders bevorzugten Ausführungsform ist der Stickstoff in 1-Position der Verbindungen der Formeln (3) und (1) unsubstituiert, d.h. der Rest R² ist Wasserstoff.

Im Rahmen der Erfindung ist es bevorzugt, dass die Reaktionsmischung, aus der das Produkt der Formel (3) mit R³ = H gewonnen werden kann, durch Zugabe von Säure oder einem Gemisch von Säuren, insbesondere Salzsäure, Schwefelsäure, Essigsäure oder Ameisensäure, in einer technisch relevanten Konzentration, sauer gestellt, anschließend das organische Lösungsmittel ganz oder teilweise abdestilliert und der Rückstand filtriert wird. Das so erhaltene feste Produkt kann mit geeigneten Lösungsmitteln gewaschen werden.

Weiterhin ist es besonders bevorzugt, dass die Reaktionsmischung aus der das Produkt der Formel (3) mit R³ = H gewonnen werden kann, durch Zugabe von Salzsäure oder Schwefelsäure einer technisch relevanten Konzentration, Essigsäure oder Ameisensäure sauer gestellt, und zusätzlich ein weiteres organisches Lösungsmittel zugeben und der Rückstand filtriert wird. Das so erhaltene feste Produkt kann mit geeigneten Lösungsmitteln gewaschen werden.

Es liegt im Rahmen der Erfindung, dass zur Reaktionsmischung, welche Produkte der Formel (3) enthält, vor oder beim Ansäuern eine geeignete Menge Entschäumer zugegeben wird, um unerwünschtes Aufschäumen der Reaktionsmischung zu vermindern.

In einer besonders bevorzugten Ausführungsform stehen die Reste R⁴ und R⁵ in den Formeln (2) und (3) unabhängig voneinander jeweils für unsubstituiertes (C₁-C₄)-Alkyl, und unsubstituiertes (C₁-C₄)-Alkoxy.

Ganz besonders bevorzugt sind Verbindungen der Formeln (2) und (3), in welchen die Reste R⁴ und R⁵ jeweils unabhängig voneinander für Methoxy, Ethoxy, Methyl, Ethyl stehen.

In einer besonders bevorzugten Ausführungsform ist die Abgangsgruppe X ein Chlor.

Ein wichtiger Aspekt betrifft die Auswahl des Lösungsmittels in dem die Umsetzung durchgeführt wird. Die Umsetzung kann in
- einem polaren, oder
- einem unpolaren Lösungsmittel, oder in
- einem Gemisch aus einem polaren oder unpolaren Lösungsmittel durchgeführt werden.

Als unpolare Lösungsmittel können
- Haloalkane, insbesondere Dichlormethan oder Dichlorethan; oder
- Aromaten, insbesondere Toluol, Xylol oder Chlorbenzol,
   verwendet werden.

Als polare organische Lösungsmittel können
- Ketone, insbesondere Aceton, Butanon, 2-Methylbutanon;
- Nitrile, insbesondere Acetonitril, Butyronitril, Isobutylnitril;
- Amide, insbesondere N,N-Dimethylformamid, N,N-Dimethylacetamid, Formamid, N-Methylformamid, N-Methylpyrrolidon;
- Sulfoxide und Sulfone, zum Beispiel Dimethylsulfoxid, Dimethlysulphone, Sulfolan;
- Ether, insbesondere Dioxan, 2-Methyltetrahydrofuran, Methylcyclopentylether, tert-Butylmethylether oder Tetrahydrofuran; oder
- Ester, insbesondere Ethylacetat, n-Butylacetat oder Isopropylacetat verwendet werden.

Die genannten polaren Lösungsmittel können entweder allein oder in Mischungen mit anderen Lösungsmitteln, bevorzugt mit weiteren polaren organischen Lösungsmitteln oder mit Wasser, eingesetzt werden. Dabei ist nicht ausgeschlossen, dass die Umsetzung auch in Wasser als einzigem Lösungsmittel erfolgt.

Besonders bevorzugt ist die Durchführung des Verfahrens ohne die Verwendung von Wasser als Lösungsmittel.

Das erfindungsgemäße Verfahren zur Herstellung von Verbindungen der Formel (3), basiert darauf, dass das Oxindol (1 Äquivalent) in einem geeigneten Lösungsmittel mit der Triazinkomponente und der Base umgesetzt wird. Dabei wird die Triazinkomponente bevorzugt im Überschuss (1.1 bis 1.4 Äquivalente, bevorzugt 1.1 bis 1.25 Äquivalente) eingesetzt.

Die Base wird äquimolar oder im Überschuss eingesetzt. Wenn R² = H wird die Base mit 2 bis 3 Äquivalenten bevorzugt mit 2.2 bis 2.6 Äquivalenten eingesetzt.

Alle Reaktanden können entweder in reiner Form oder miteinander vorgemischt oder in einem Lösungsmittel oder einem Lösungsmittelgemisch gelöst oder suspendiert zur Reaktionsmischung gegeben werden.

Für gute Produktausbeuten hat sich gezeigt, dass es vorteilhaft sein kann, zuerst das Oxindol mit der Base (Gesamtmenge oder Teilmenge) in einem geeigneten Lösungsmittel zur Reaktion zu bringen, und anschließend die Triazinkomponente und gegebenenfalls eine weitere Menge derselben oder einer anderen Base oder einer Mischung verschiedener Basen in einer oder mehreren Portionen zuzugegeben

Eine andere Zugabevariante besteht darin, dass das Oxindol und die Triazinkomponente in einem geeigneten Lösungsmittel vorgelegt werden und die Base, bzw. die Mischung verschiedener Basen portionsweise zugegeben wird.

Die Zugabe der Reaktanden kann in einer Portion oder in mehreren Portionen über einen Zeitraum von bis zu 24 Stunden, bevorzugt bis zu 6 Stunden, insbesondere 0,05 bis 6 Stunden, erfolgen.

Die Reaktionstemperatur liegt im Bereich von -20°C bis 150°C, bevorzugt im Bereich -10°C bis 90°C.

Gegebenenfalls kann die Reaktion unter Druck durchgeführt werden.

Im Verlauf der Reaktion kann weiteres Lösungsmittel zugegeben werden, um eine bessere Durchmischung der Reaktanden zu ermöglichen.

Abhängig von den verwendeten Reaktionsbedingungen liegt die Nachrührzeit nach Zugabe aller Reaktanden im Bereich bis zu 48 Stunden, bevorzugt 0,05 bis 24 Stunden.

Aufarbeitung und Isolierung des gewünschten Produktes der Formel (3) kann auf verschiedene Weisen erfolgen, beispielsweise abhängig davon, welches Lösungsmittel verwendet wird oder ob es sich bei dem Produkt um einen Feststoff oder um eine Flüssigkeit handelt.

Gegenstand der Erfindung sind auch die Verbindungen der Formel (3), 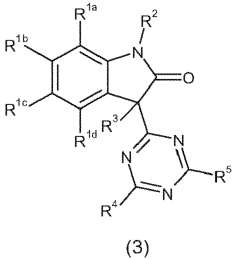 welche beispielsweise durch das oben beschriebene erfindungsgemäße Verfahren erhältlich sind, und deren Salze (3"), 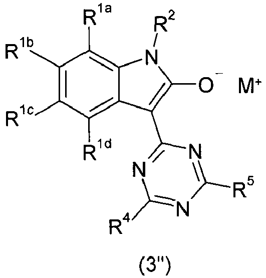 worin jeweils
R^{1a} bis R^{1d} unabhängig voneinander ausgewählt sind aus der Gruppe bestehend aus Wasserstoff, Fluor, Chlor, Brom, Iod sowie aus
(C₁-C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₈)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₁-C₆)-Alkylthio, wobei der Alkylthiorest verzweigt oder unverzweigt ist und unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der Fluor, Chlor, (C₁-C₄)-Alkyl oder (C₁-C₄)-Alkoxy bestehenden Gruppe, substituiert ist, und Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, lod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R² für
Wasserstoff,
(C₁-C₆)-Alkyl, wobei der Alkylrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist, oder
Benzyl, wobei das Benzyl unsubstituiert ist oder substituiert ist durch einen oder mehrere Substituenten, ausgewählt aus der Gruppe bestehend aus Fluor, Chlor, Brom, lod, Nitro, (C₁-C₄)-Alkyl, (C₁-C₄)-Alkoxy oder (C₃-C₇)-Cycloalkyl oder (C₁-C₄)-Alkylthio sowie aus COOR^{a}, worin R^{a} für ein (C₁-C₄)-Alkyl steht, und -CONR^{b'}R^{b"} oder-CONHR^{b"} worin R^{b'} und R^{b"} jeweils unabhängig voneinander für ein (C₁-C₄)-Alkyl steht, wobei jeweils zwei Substituenten am N-Atom zusammen gegebenenfalls einen unsubstituierten oder substituierten Ring bilden,
steht,
R³ für
Wasserstoff oder Methyl
steht,
R⁴ und R⁵ unabhängig voneinander jeweils für
Wasserstoff,
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist, (C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
stehen,
wobei in den Salzen der allgemeinen Formel (3")
M für Li, Na, K, N(R^{c})₄, mit R^{c} = H oder C₁-C₆ Alkyl, Cs, Ba, Mg, Ca und Zn steht und die Anzahl der Gegenionen M⁺ sich nach der jeweiligen Ladung richtet, so dass eine insgesamt neutrale Verbindung der allgemeinen Formel (3") entsteht.

Von den Formeln (3) und (3") sind, soweit zutreffend, auch alle Stereoisomeren, Tautomeren und/oder polymorphe Formen sowie deren Salze umfasst.

Besonders bevorzugt sind Verbindungen der allgemeinen Formeln (3), in denen R³ für H oder Methyl steht.

Am meisten bevorzugt sind Verbindungen der allgemeinen Formeln (3), in denen R³ für H steht.

Verbindungen der allgemeinen Formeln (3) und (3") sowie die nach dem erfindungsgemäßen Verfahren hergestellten Verbindungen der allgemeinen Formel (3) eignen sich als Zwischenprodukte zur Herstellung von Feinchemikalien und Wirkstoffen aus der Landwirtschaft.

Verbindungen der Formel (3) und (3") sind triazinylsubstituierte Oxindole. Im nachfolgenden Schema 3 sind triazinylsubstituierte Oxindole mit der Formel (5-1) bezeichnet.

Schema 3 zeigt ein neues mehrstufiges Syntheseverfahren, gemäß dem, ausgehend von einem 3-(Alkylsulfanyl)-1,3-dihydro-2H-indol-2-on der Formel (7-1) in einer insgesamt fünfstufigen Reaktion ein N-Alkyl-N-[2-(1,3,5-triazin-2-ylcarbonyl)phenyl]alkansulfonamid der Formel (4-1), dessen herbizide (siehe WO 2007/031208 A2) und fungizide (siehe WO 2006/008159 A1) Aktivität bereits seit längerem bekannt ist, hergestellt werden kann.

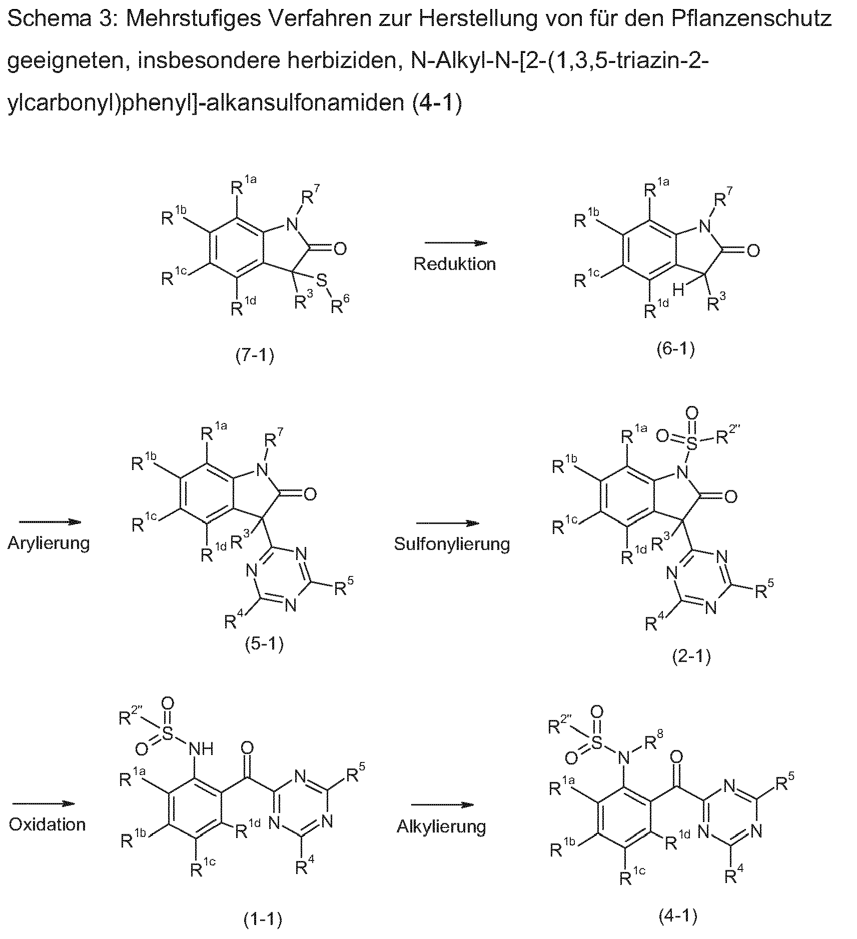

Das mehrstufige Verfahren zur Herstellung von N-Alkyl-N-[2-(1,3,5-triazin-2-ylcarbonyl)phenyl]alkansulfonamiden (4-1) besteht aus folgenden Teilschritten:
- Reduktion von substituierten oder unsubstituierten 3-(Alkylsulfanyl)-1,3-dihydro-2H-indol-2-onen (7-1) zu substituierten oder unsubstituierten 1,3-Dihydro-2H-indol-2-onen (6-1). Dieses Verfahren ist im technischen Maßstab möglich und ist in der Patentanmeldung mit der Anmeldenummer EP 10162381.7 beschrieben.
- Arylierung von substituierten oder unsubstituierten 1,3-Dihydro-2H-indol-2-onen (6-1) zu triazinylsubstituierten Oxindolen (5-1). Dieses Verfahren ist im technischen Maßstab möglich und ist in der vorliegenden Patentanmeldung beschrieben.
- Sulfonylierung von triazinylsubstituierten Oxindolen (5-1) zu N-sulfonylsubstituierten 3-Triazinyloxindolen (2-1). Dieses Verfahren ist im technischen Maßstab möglich und ist in der Patentanmeldung mit der Anmeldenummer EP 111598751 beschrieben.
- Oxidative Ringöffnung von N-sulfonylsubstituierten 3-Triazinyloxindolen (2-1) zu 2-(Triazinylcarbonyl)sulfonaniliden (1-1). Dieses Verfahren ist im technischen Maßstab möglich und ist in der Patentanmeldung mit der Anmeldenummer DE 102011086382.6 beschrieben.
- Alkylierung von 2-(Triazinylcarbonyl)sulfonaniliden (1-1) zu N-Alkyl-N-[2-(1,3,5-triazin-2-ylcarbonyl)phenyl]alkansulfonamiden (4-1). Dieses Verfahren ist in der Patentanmeldung mit der Anmeldenummer WO 2006/008159 beschrieben.

Das in Schema 3 dargestellte neue mehrstufige Verfahren zeichnet sich gegenüber den vorbekannten Verfahren zur Herstellung von N-Alkyl-N-[2-(1,3,5-triazin-2-ylcarbonyl)phenyl]-alkansulfonamiden (4-1) sowie 2-(Triazinylcarbonyl)sulfonaniliden (1-1) dadurch aus, dass Oxindolverbindungen als Edukte bzw. als Intermediate eingesetzt werden. Dies bringt den Vorteil, dass es im Vergleich zu den vorbekannten Verfahren im technischen Maßstab durchgeführt werden kann und zugleich hohe Ausbeuten erhalten werden können.

Die Ausführbarkeit des in Schema 3 zusammengefassten Verfahrens ist im Detail nachfolgend offenbart. Die Reduktion, die in Schema 3 den ersten Reaktionsschritt des insgesamt fünfstufigen Verfahrens betrifft, wurde nachfolgend als eigenständige Vorstufe B) behandelt. Das nachfolgend im Detail beschriebene Verfahren A) umfasst somit die Schritte der Arylierung, der Sulfonylierung, der Oxidation und der Alkylierung.
A)Verfahren zur Herstellung von N-Alkyl-N-[2-(1,3,5-triazin-2-ylcarbonyl)phenyl]alkansulfonamiden der Formel (4-1) 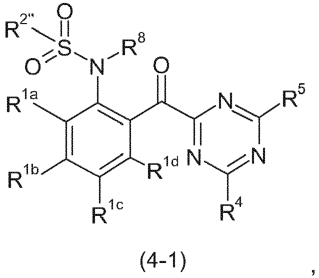 worin
   R^{1a} bis R^{1d} unabhängig voneinander ausgewählt sind aus der Gruppe bestehend aus Wasserstoff, Fluor, Chlor, Brom, lod sowie aus
   (C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
   (C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
   (C₁-C₆)-Alkoxy, wobei der Alkoxyrest unsubstituiert ist oder durch einen oder mehrere Substituenten, ausgewählt aus der aus Fluor, Chlor, (C₁-C₄)-Alkoxy
   oder (C₃-C₇)-Cycloalkyl bestehenden Gruppe, substituiert ist, (C₃-C₇)-Cycloalkoxy, wobei der Cycloalkoxyrest unsubstituiert ist oder durch einen
   oder mehrere Substituenten, ausgewählt aus der aus Fluor, Chlor, (C₁-C₄)-Alkyl oder (C₁-C₄)-Alkoxy bestehenden Gruppe, substituiert ist, (C₁-C₆)-Alkylthio, wobei der Alkylthiorest unsubstituiert ist oder durch einen oder mehrere Substituenten, ausgewählt aus der aus Fluor, Chlor, (C₁-C₄)-Alkyl
   oder (C₁-C₄)-Alkoxy bestehenden Gruppe, substituiert ist, (C₃-C₇)-Cycloalkylthio, wobei der Cycloalkylthiorest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist, und Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, lod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
   R^{2"} für
   (C₁-C₆)-Alkyl, wobei der Alkylrest unsubstituiert ist oder ganz oder teilweise mit Fluor substituiert ist, oder
   (C₃-C₇)-Cycloalkyl, wobei der Cycloalkylrest unsubstituiert ist oder ganz oder teilweise mit Fluor substituiert ist,
   R⁴ und R⁵ unabhängig voneinander jeweils für
   Wasserstoff,
   (C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
   (C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
   stehen, und
   R⁸ für
   (C₁-C₆)-Alkyl, wobei der Alkylrest unsubstituiert ist oder ganz oder teilweise mit Fluor substituiert ist,
   (C₁-C₆)-Cycloalkyl, (C₁-C₆)-Alkenyl oder (C₁-C₆)-Alkoxyalkyl, wobei jeder der genannten Reste unsubstituiert ist oder ganz oder teilweise mit Fluor substituiert ist,
   steht, wobei
   ein 1,3-Dihydro-2H-indol-2-on der Formel (6-1) 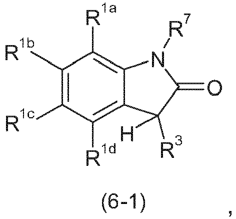 worin
   R^{1a} bis R^{1d} wie für Formel (4-1) definiert sind,
   R³ für Wasserstoff steht, und
   R⁷ für Wasserstoff steht, in einem
   ersten Schritt durch
   - Arylierung zu einem triazinylsubstituierten Oxindol der Formel (5-1) 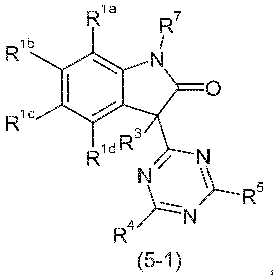 worin
      R^{1a} bis R^{1d} und R⁴ und R⁵ wie für die Formel (4-1) und R³ und R⁷ wie für die Formel (5-1) definiert sind,
      umgesetzt wird, und die Arylierungsprodukte der Formel (5-1) in einem
      zweiten Schritt durch
   - Sulfonylierung zu N-sulfonylsubstituierten 3-Triazinyloxindolen der Formel (2-1) 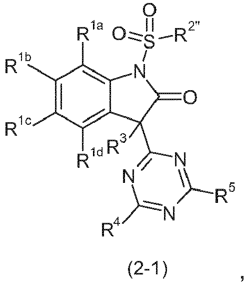 worin R^{1a} bis R^{1d}, R² sowie R⁴ und R⁵ wie in Formel (4-1) und R³ wie für die Formel (5-1)definiert sind,
      umgesetzt werden, und die Sulfonylierungsprodukte der Formel (2-1) in einem
      dritten Schritt durch
   - oxidative Ringöffnung zu einem 2-(Triazinylcarbonyl)sulfonanilid der Formel (1-1) 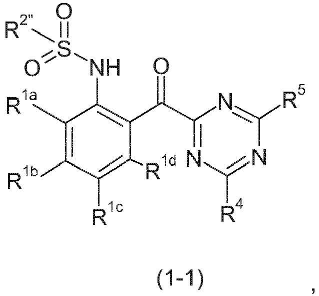 worin
      R^{1a} bis R^{1d}, R^{2"} sowie R⁴ und R⁵ wie für Formel (4-1) definiert sind,
      umgesetzt werden, und die Oxidationsprodukte der Formel (1-1) in einem
      vierten Schritt durch
   - Alkylierung zu einem N-Alkyl-N-[2-(1,3,5-triazin-2-ylcarbonyl)phenyl] alkansulfonamid der Formel (4-1) 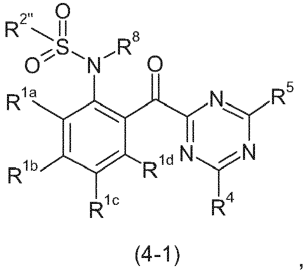 worin
      R^{1a} bis R^{1d}, R^{2"} , R⁴, R⁵ und R⁸ wie oben für Formel (4-1) definiert sind, umgesetzt werden, wobei als Alkylierungsreagenz
   - X- R⁸, wobei X für Chlor, Brom oder lod steht und R⁸ wie oben für Formel (4-1) definiert ist, oder
   - (R⁸)₂SO₄, worin R⁸ wie oben für Formel (4-1) definiert ist,
      eingesetzt wird.

   Die Sulfonylierung erfolgt in Gegenwart
   - einer in 1-Position substituierten Imidazol-Base, oder
   - eines Basen-Gemischs, das mindestens eine in 1-Position substituierte Imidazol-Base enthält.

   Besonders bevorzugte Imidazol-Basen sind 1-Methyl-1H-Imidazol, 1-Butyl-1H-Imidazol oder 1-Benzyl-1H-Imidazol, die einzeln oder im Gemisch eingesetzt werden können, wobei die Verwendung von 1-Methyl-1H-Imidazol ganz besonders bevorzugt ist.
B) Verfahren zur Herstellung von N-Alkyl-N-[2-(1,3,5-triazin-2-ylcarbonyl)phenyl] alkansulfonamiden der Formel (4-1) bei dem die als Edukt eingesetzten Verbindungen der Formel (6-1) in einem dem Verfahren zur Herstellung von Verbindungen der Formel (4-1) vorhergehenden Verfahrensschritt hergestellt werden, wobei ausgehend von einem 3-(Alkylsulfanyl)-1,3-dihydro-2H-indol-2-on der Formel (7-1), 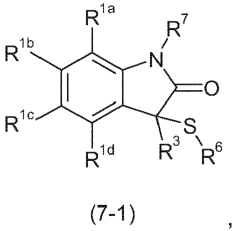 worin
   R^{1a} bis R^{1d} wie für Formel (4-1) definiert sind,
   R³ für Wasserstoff steht,
   R⁷ für Wasserstoff steht, und
   R⁶ ein unsubstituiertes oder substituiertes (C₁-C₁₄)-Alkyl, (C₃-C₇)-Cycloalkyl, Benzyl oder ein CH₂-C(O)O-(C₁-C₆)-Alkyl ist,
   durch
   - Reduktion zu einem 1,3-Dihydro-2H-indol-2-on (6-1) 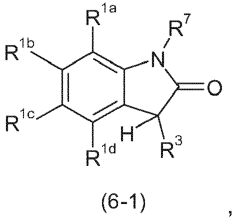 worin
      R^{1a} bis R^{1d}, R³ und R⁷ wie für Formel (7-1) definiert sind,
      umgewandelt wird.

Bei der Reduktion wird
a) eine Verbindung der Formel (7-1) in einem polaren Lösungsmittel gelöst oder suspendiert wird,
b) der Lösung oder der Suspension ein schwefelhaltiges Salz zugesetzt wird, und
c) das Reaktionsgemisch bei einer Temperatur, die maximal der Siedetemparatur des polaren Lösungsmittels entspricht, unter Rückfluss erwärmt wird.

Als schwefelhaltige Salze besonders bevorzugt sind Natriumsalze, ausgewählt aus der Gruppe bestehend aus Natriumbisulfit, Natriumsulfit, Natriumthionit, Natriumdithionit und Natriumthiosulfat.

Die herbizide (siehe WO 2007/031208 A2) und fungizide (siehe WO 2006/008159 A1) Wirkung von N-Alkyl-N-[2-(1,3,5-triazin-2-ylcarbonyl)phenyl]alkansulfonamiden der Formel (4-1) ist, wie bereits erwähnt, seit längerem bekannt.

Somit wird durch Schema 3 und die Verfahren A) und B) belegt, dass triazinylsubstituierte Oxindole der Formel (3) als Intermediate zur Herstellung von Pflanzenschutzmitteln, insbesondere von Herbiziden und Fungiziden, geeignet sind.

Gegenstand der Erfindung ist daher auch die Verwendung der erfindungsgemäß hergestellten Verbindungen der Formeln (3) oder deren Salze (3") zur Herstellung von Wirkstoffen aus der Landwirtschaft oder von Zwischenprodukten zur Herstellung von Feinchemikalien und Wirkstoffen aus der Landwirtschaft, besonders Pflanzenschutzmitteln.

Bevorzugt ist die Verwendung von Verbindungen der Formeln (3) oder deren Salze (3") als Zwischenprodukte zur Herstellung von N-[2-(1,3,5-Triazin-2-ylcarbonyl)phenyl]alkylsulfonamiden.

### BEISPIELE

Die nachfolgenden Beispiele erläutern die Erfindung näher.

In den nachfolgenden Beispielen beziehen sich Mengenangaben auf das Gewicht, sofern nichts anderes speziell definiert ist (in der Beschreibung wurde hierfür analog Gew.% = Gewichtsprozent verwendet). Für Maßeinheiten, physikalische Größen und ähnliches werden übliche Abkürzungen verwendet, beispielsweise h = Stunde(n), mpt = Schmelzpunkt (Smp.), l = Liter, ml = Milliliter, g = Gramm, min = Minute(n), *in vacuo* = "im Vakuum" = unter reduziertem Druck, d. Th. = Prozent Ausbeute nach der Theorie.

### Beispiel 1:

### Herstellung von 3-(4,6-Dimethoxy-1,3,5-triazin-2-yl)-7-fluor-1,3-dihydro-2H-indol-2-on

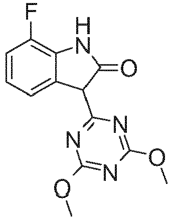

### Variante A:

7-Fluor-1,3-dihydro-2H-indol-2-on (100g) wird in 600 ml N,N-Dimethylacetamid vorgelegt und unter Eis/Methanol-Kühlung auf ca. 0°C gekühlt. Eine Lösung von Kaliumhydroxid (43,2g) und Kaliumcarbonat (143g) in 600 ml Wasser wird zugegeben und kurz nachgerührt. Anschließend wird 2-Chlor-4,6-dimethoxy-1,3,5-triazin (140,8g) zugegeben und mit je 100 ml Wasser und N,N-Dimethylacetamid nachgespült. Das Kühlbad wird entfernt, der Ansatz erwärmt sich auf ca. 30°C. Es wird 18 Stunden bei Raumtemperatur gerührt. Nach Zugabe 150 ml Toluol wird mit verdünnter Salzsäure (ca. 700 ml) wird auf pH 3-4 gestellt und dabei etwas Entschäumer (Fluowet PL 80) zugegeben. Der Feststoff wird abgesaugt, dreimal mit je 250 ml Wasser und zweimall mit je 250 ml Heptan gewaschen und im Vakuum bei 55°C getrocknet. Man erhält die Titelverbindung als Feststoff in einer Reinheit von 96,6% (187,5g, 95% d. Th).
LC-MS: M+H = 291 (100%).
1H-NMR (400 MHz, DMSO-D₆): δ (ppm) = 11.37 (s, 1H), 7.64 (d, 1H), 6.97 (dt, 1H), 6.86 (dd, 1H), 4.05 (s, 6H).

### Variante B:

7-Fluor-1,3-dihydro-2H-indol-2-on (50g) wird in 400 ml Aceton vorgelegt und Kaliumhydroxid (21,5g) zugegeben. Anschließend gibt man 2-Chlor-4,6-dimethoxy-1,3,5-triazin (70,06g) hinzu spült mit 100 ml Aceton nach. Die Reaktionsmischung wird eine Stunde bei Rückfluss gerührt, das Wärmebad entfernt und Kaliumhydroxid (21,5g) portionsweise zugegeben. Anschließend wird noch 2 Stunden bei Rückfluss gerührt. Der Ansatz auf 25°C abgekühlt, zehnprozentige Salzsäure (140 ml) zugegeben, mit 250 ml Wasser verdünnt und eine Stunde nachgerührt. Der Feststoff wird abgesaugt, zweimal mit je 100 ml Wasser/Aceton (3:1) gewaschen und im Vakuum bei 50°C getrocknet. Man erhält die Titelverbindung als Feststoff in einer Reinheit von 96,2% (80,09g, 81% d. Th). Die NMR-Signale des Produktes stimmen mit den Signalen des nach Variante A erhaltenen Produktes überein.

### Variante C:

7-Fluor-1,3-dihydro-2H-indol-2-on (12g) und 2-Chlor-4,6-dimethoxy-1,3,5-triazin (16,5g) werden bei Raumtemperatur in 160ml Formamid vorgelegt und Kaliumcarbonat (24,3g) in drei gleichen Portionen innerhalb 1,5 Stunden zugegeben. Die Reaktionsmischung wird 4 - 5 Stunden bei Raumtemperatur nachgerührt. Der Ansatz wird auf 500 ml Wasser gegeben und mit verdünnter Salzsäure auf pH 3 gestellt. Der Feststoff wird abgesaugt und mit Wasser, anschließend mit Acetonitril, gewaschen und im Vakuum getrocknet. Man erhält die Titelverbindung als Feststoff in einer HPLC-Reinheit von 98 % Fläche (93,0g, 80% d. Th). Die NMR-Signale des Produktes stimmen mit den Signalen des nach Variante A erhaltenen Produktes überein.

### Variante D:

7-Fluor-1,3-dihydro-2H-indol-2-on (60g) und 2-Chlor-4,6-dimethoxy-1,3,5-triazin (94,6g) werden in 315 ml THF vorgelegt und auf 5°C gekühlt. Unter Eiskühlung wird eine Lösung von Kaliumhydroxid (58,1g) in 105 ml Wasser innerhalb 2 Stunden bei einer Innentemperatur von 0-15°C zugegeben und 4 Stunden nachgerührt. Der Feststoff wird abgesaugt, mit Wasser (zweimal 150 ml) gewaschen und im Vakuum getrocknet. Man erhält die Titelverbindung als Feststoff in einer Reinheit von 98,2 % (21,1g, 91% d. Th). Die NMR-Signale des Produktes stimmen mit den Signalen des nach Variante A erhaltenen Produktes überein.

### Variante E:

7-Fluor-1,3-dihydro-2H-indol-2-on (30 g) und 2-Chlor-4,6-dimethoxy-1,3,5-triazin (43,9 g) werden bei 60°C in Acetonitril vorgelegt und Natriumhydroxid (16,8 g) in vier gleichen Portionen innerhalb 40 min zugegeben. Es wurde noch 90 min bei dieser Temperatur nachgerührt und dann auf 40°C abgekühlt. Man gab Salzsäure (20%ig, 42 g) und Wasser (160 g) zu. Nach weiteren 30 min wurde die entstandene Suspension filtriert und der Filterrückstand mit Acetonitril gewaschen. Nach Trocknen im Vakuum (50°C, <200 mbar) erhielt man die Titelverbindung als Feststoff mit einer HPLC-Reinheit von 96,9% (48,0 g; 85% d. Th). Die NMR-Signale des Produktes stimmen mit den Signalen des nach Variante A erhaltenen Produktes überein.

### Variante F:

7-Fluor-1,3-dihydro-2H-indol-2-on (10g; 1 eq.) wird in 100 ml THF und 100 ml N,N-Dimethylformamid bei Raumtemperatur unter Stickstoff vorgelegt und Natriumhydrid (2,63g; 60% in Mineralöl, 1 eq.) zugegeben. Man rührt 30 min nach und gibt 2-Chlor-4,6-dimethoxy-1,3,5-triazin (4,72g; 0,4 eq.) in einer Portion zu. Es wird 10 min bei 35°C und 2 Stunden bei 80°C nachgerührt. Der Ansatz wird abgekühlt und im Vakuum eingeengt (50°C Badtemperatur, 10 mbar). Zum Rückstand wird 200 ml Wasser gegeben und mit Salzsäure auf pH 3-4 gestellt. Der ausgefallene Feststoff wird abgesaugt, das Filtrat enthält nach HPLC praktisch kein Produkt. Der Filterrückstand wird mit Wasser (50 ml) gewaschen, wasserfeucht mit 75 ml Acetonitril ausgerührt, abgesaugt, mit Acetonitril nachgewaschen und im Vakuum getrocknet. Man erhält die Titelverbindung als Feststoff in einer HPLC-Reinheit von 97 % Fläche (7,62g; 39% d. Th. bezogen auf das eingesetzte 7-Fluor-1,3-dihydro-2H-indol-2-on bzw. 97% d.Th. bezogen auf das eingesetzte 2-Chlor-4,6-dimethoxy-1,3,5-triazin). Die NMR-Signale des Produktes stimmen mit den Signalen des nach Variante A erhaltenen Produktes überein.

### Variante G:

7-Fluor-1,3-dihydro-2H-indol-2-on (10g; 1 eq.) und 2-Chlor-4,6-dimethoxy-1,3,5-triazin (14,1g; 1,2 eq.) werden analog zu Beispiel 1 Variante F umgesetzt. Man erhält die Titelverbindung als Feststoff in einer HPLC-Reinheit von 97 % Fläche (7,65g; 39% d. Th. bezogen auf das eingesetzte 7-Fluor-1,3-dihydro-2H-indol-2-on). Die NMR-Signale des Produktes stimmen mit den Signalen des nach Variante A erhaltenen Produktes überein.

### Variante H (Durchführung analog zu Organic Letters (2010) 2306 Beispiele in Table 4 (Supplement Seite S-12 General Procedure):

7-Fluor-1,3-dihydro-2H-indol-2-on (2,5g; 1 eq.), 2-Chlor-4,6-dimethoxy-1,3,5-triazin (3,5g; 1,2 eq.) und Cäsiumcarbonat (5,6g, 1 eq.) werden unter Stickstoff vorgelegt und 95 ml N,N-Dimethylformamid (Wassergehalt < 0,1 %) hinzugegeben. Der Ansatz wird auf 65°C erwärmt und 5 Stunden bei 65°C unter Stickstoff gerührt. Eine HPLC-Analytik (Detektion bei 210 nm, Angaben in Flächenprozent, Lösungsmittelsignal wird nicht integriert) zeigt 16% 7-Fluor-1,3-dihydro-2H-indol-2-on, 19% der Titelverbindung (3-(4,6-Dimethoxy-1,3,5-triazin-2-yl)-7-fluor-1,3-dihydro-2H-indol-2-on), 57% einer Hauptnebenkomponente (oder einer Mischung mehrerer Hauptnebenkomponenten) und weitere kleine Nebenkomponenten. Der Ansatz wird auf Raumtemperatur gekühlt und auf 200 ml gesättigte Ammoniumchlorid-Lösung gegeben. Es wird Ethylacetat zugegeben, bis sich zwei klare Phasen bilden (insgesamt 2900 ml), die Phasen getrennt, die organische Phase mit Wasser (2 mal 150 ml) und 150 ml Natriumchloridlösung gewaschen und über Magnesiumsulfat getrocknet. Das Lösungsmittel wird im Vakuum entfernt, wobei ein Feststoff ausfällt. Der Feststoff wird abfiltriert und mit etwas Ethylacetat gewaschen. Man erhält 1,92 g einer Mischung, welche nach HPLC-Analytik (Detektion bei 210 nm, Angaben in Flächenprozent) zu 56% (22% d. Th.) aus der Titelverbindung und zu 43% aus einer Nebenkomponente besteht. Ein LC-MS der Mischung zeigt das Vorhandensein der Titelverbindung (M+H = 291) und einer Nebenkomponente der Masse von 429 (M+H = 430), bei der es sich wahrscheinlich um ein zweifach aryliertes Produkt handelt. Die Identität der in der Produktmischung enthaltenen Titelverbindung kann durch Aufstocken der Reaktionsmischung mit authentischem Material, hergestellt nach Beispiel 1 Variante A, und Vergleich der UV-Absorptionen, sowie durch NMR-Spektroskopie der Mischung bestätigt werden.

Das Filtrat wird im Vakuum eingeengt, man erhält 3.6 g Rückstand, welcher noch DMF enthält. Eine HPLC-Analytik (Detektion bei 210 nm, Angaben in Flächenprozent, Lösungsmittelsignal wird nicht integriert) des Rückstandes zeigt 19% 7-Fluor-1,3-dihydro-2H-indol-2-on, 1% der Titelverbindung, 68% einer Mischung aus zwei Hauptnebenkomponenten und weitere kleine Nebenkomponenten. Ein LC-MS des Rückstandes zeigt das Vorhandensein der Titelverbindung (M+H = 291, 4%) und zwei Nebenkomponente der Masse von 429 (M+H = 430, 19% und 49%), bei denen es sich vermutlich um zweifach arylierte Produkte handelt.

### Beispiel 2:

### Herstellung von 3-(4,6-Dimethoxy-1,3,5-triazin-2-yl)-1,3-dihydro-2H-indol-2-on

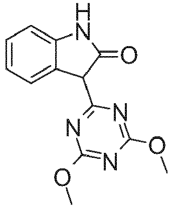

### Variante A:

1,3-Dihydro-2H-indol-2-on (3,0g) und 2-Chlor-4,6-dimethoxy-1,3,5-triazin (7,52g) werden analog zu Beispiel 1 Variante C umgesetzt. Man erhält die Titelverbindung als Feststoff in einer HPLC-Reinheit von 91 % Fläche (4,79g, 73% d. Th).
LC-MS: M+H = 273 (91,8%).
1H-NMR (400 MHz, DMSO-D₆): δ (ppm) = 10.96 (s, 1H), 7.78-7.84 (m, 1H), 6.95-7.04 (m, 3H), 4.04 (s, 6H).

### Variante B:

1,3-Dihydro-2H-indol-2-on (0,80g; 1 eq.) wird in 8 ml THF und 8 ml N,N-Dimethylformamid bei Raumtemperatur unter Stickstoff vorgelegt und Natriumhydrid (0,24g; 60% in Mineralöl, 1 eq.) zugegeben. Man rührt 30 min nach und gibt 2-Chlor-4,6-dimethoxy-1,3,5-triazin (0,42g; 0,4 eq.) in einer Portion zu. Es wird 2 Stunden bei Raumtemperatur nachgerührt. Der Ansatz wird abgekühlt und im Vakuum eingeengt (40°C Badtemperatur). Zum Rückstand wird 25 ml Wasser gegeben und mit Salzsäure auf pH 3-4 gestellt. Der ausgefallene Feststoff wird abgesaugt, das Filtrat enthält nach HPLC praktisch kein Produkt. Der Filterrückstand wird mit Wasser gewaschen, wasserfeucht mit 10 ml Acetonitril ausgerührt und abgesaugt. Man erhält die Titelverbindung als Feststoff in einer HPLC-Reinheit von 90 % Fläche (0,61g; 34% d. Th. bezogen auf das eingesetzte 1,3-Dihydro-2H-indol-2-on bzw. 84% d.Th. bezogen auf das eingesetzte 2-Chlor-4,6-dimethoxy-1,3,5-triazin). Die NMR-Signale des Produktes stimmen mit den Signalen des nach Variante A erhaltenen Produktes überein.

### Variante C:

1,3-Dihydro-2H-indol-2-on (0,80g; 1 eq.) und 2-Chlor-4,6-dimethoxy-1,3,5-triazin (1,27g; 1,2 eq.) werden analog zu Beispiel 1 Variante F umgesetzt. Man erhält die Titelverbindung als Feststoff in einer HPLC-Reinheit von 78% Fläche (0,59g; 30% d. Th. bezogen auf das eingesetzte 1,3-Dihydro-2H-indol-2-on). Die NMR-Signale des Produktes stimmen mit den Signalen des nach Variante A erhaltenen Produktes überein.

### Beispiel 3:

### Herstellung von 3-(4,6-Dimethoxy-1,3,5-triazin-2-yl)-5,7-difluor-1,3-dihydro-2H-indol-2-on

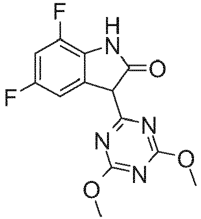

5,7-Difluor-1,3-dihydro-2H-indol-2-on (1,69g) und 2-Chlor-4,6-dimethoxy-1,3,5-triazin (2,13g) werden analog zu Beispiel 1 Variante A umgesetzt. Man erhält die Titelverbindung als Feststoff in einer HPLC-Reinheit von 93 % Fläche (2,82g, 85% d. Th).
LC-MS: M+H = 309 (97%).
1H-NMR (400 MHz, DMSO-D₆): δ (ppm) = 11.42 (s, 1H), 7.35 (dd, 1H), 6.86 (dt, 1H), 4.04 (s, 6H).

### Beispiel 4:

### Herstellung von 3-(4,6-Dimethoxy-1,3,5-triazin-2-yl)-7-chlor-1,3-dihydro-2H-indol-2-on

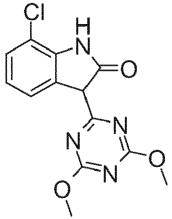

7-Chlor-1,3-dihydro-2H-indol-2-on (101,5g) und 2-Chlor-4,6-dimethoxy-1,3,5-triazin (152g) werden analog zu Beispiel 1 Variante A umgesetzt. Man erhält die Titelverbindung als Feststoff in einer HPLC-Reinheit von 99 % Fläche (181,9g, 97% d. Th).
LC-MS: M+H = 307 (96,7%).
1H-NMR (400 MHz, DMSO-D₆): δ (ppm) = 11.39 (s, 1H), 7.77 (d, 1H), 6.98-7.06 (m, 2H), 4.05 (s, 6H).

### Beispiel 5:

### Herstellung von 3-(4,6-Dimethoxy-1,3,5-triazin-2-yl)-5-fluor-1,3-dihydro-2H-indol-2-on

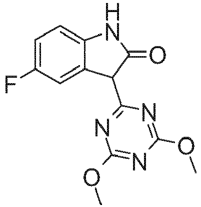

5-Fluor-1,3-dihydro-2H-indol-2-on (10g) und 2-Chlor-4,6-dimethoxy-1,3,5-triazin (15,5g) werden analog zu Beispiel 1 Variante A umgesetzt. Man erhält die Titelverbindung als Feststoff in einer HPLC-Reinheit von 92 % Fläche (19g, 91% d. Th).
LC-MS: M+H = 291 (90%).
1H-NMR (400 MHz, DMSO-D₆): δ (ppm) = 11.06 (s, 1H), 7.53 (dd, 1H), 6.95 (dd, 1H), 6.82 (dt, 1H), 4.06 (s, 6H).

### Beispiel 6:

### Herstellung von 3-(4,6-Dimethoxy-1,3,5-triazin-2-yl)-7-methoxy-1,3-dihydro-2H-indol-2-on

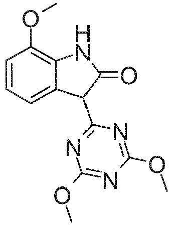

7-Methoxy-1,3-dihydro-2H-indol-2-on (1,24g) und 2-Chlor-4,6-dimethoxy-1,3,5-triazin (1,84g) werden analog zu Beispiel 1 Variante A umgesetzt. Man erhält die Titelverbindung als Feststoff in einer HPLC-Reinheit von 87 % Fläche (1,04g, 43% d. Th).
LC-MS: M-H = 301 (84%).
1H-NMR (400 MHz, DMSO-D₆): δ (ppm) = 11.36 (s, 1H), 7.52 (d, 1H), 6.96 (t, 1H), 6.71 (d, 1H), 4.04 (s, 6H), 3.85 (s, 3H).

### Beispiel 7:

### Herstellung von 3-(4,6-Dimethoxy-1,3,5-triazin-2-yl)-5-methoxy-1,3-dihydro-2H-indol-2-on

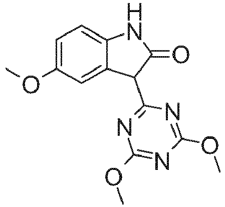

5-Methoxy-1,3-dihydro-2H-indol-2-on (1,59g) und 2-Chlor-4,6-dimethoxy-1,3,5-triazin (1,97g) werden analog zu Beispiel 1 Variante A umgesetzt. Man erhält die Titelverbindung als Feststoff in einer HPLC-Reinheit von 92 % Fläche (1,39g, 53% d. Th).
LC-MS: M+H = 303 (94%).
1H-NMR (400 MHz, DMSO-D₆): δ (ppm) = 10.89 (s, 1H), 7.43 (d, 1H), 6.89 (d, 1H), 6.61 (dd, 1H), 4.06 (s, 6H), 3.74 (s, 3H).

### Beispiel 8:

### Herstellung von 7-Fluor-3-(4-methoxy-6-methyl-1,3,5-triazin-2-yl)-1,3-dihydro-2H-indol-2-on

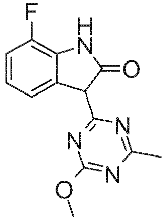

7-Fluor-1,3-dihydro-2H-indol-2-on (3,05g) und 2-Chlor-4-methoxy-6-methyl-1,3,5-triazin (6,59g) werden analog zu Beispiel 1 Variante A umgesetzt. Man erhält die Titelverbindung als Feststoff in einer HPLC-Reinheit von 85 % Fläche (4,73g, 73% d. Th).
LC-MS: M+H = 275 (72%).
1H-NMR (400 MHz, DMSO-D₆): δ (ppm) = 11.02 (s, 1H), 7.59 (d, 1H), 6.91-6.98 (m, 1H), 6.85 (t, 1H), 4.06 (s, 3H), 2.46 (s, 3H).

### Beispiel 9:

### Herstellung von 3-(4,6-Diethoxy-1,3,5-triazin-2-yl)-7-fluor-1,3-dihydro-2H-indol-2-on

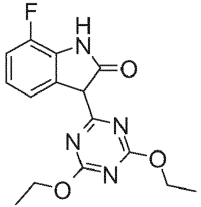

7-Fluor-1,3-dihydro-2H-indol-2-on (1,0g) wird in 10 ml N,N-Dimethylacetamid vorgelegt. Eine Lösung von Kaliumcarbonat (1,8g) und Kaliumhydroxid (0,3g) in 10 ml Wasser wird zugegeben und der Ansatz kurz nachgerührt. Anschließend werden eine Lösung von 2-Chlor-4,6-diethoxy-1,3,5-triazin (3,0g, ca. 50% Reinheit) in 10 ml N,N-Dimethylacetamid sowie 10 ml Wasser zugegeben. Aus der klaren gelbe Lösung scheidet sich ein Feststoff ab. Der Ansatz wird bei Raumtemperatur gerührt und weiteres 2-Chlor-4,6-diethoxy-1,3,5-triazin (1g, ca. 50% Reinheit) in zwei Portionen nach 2 bzw. 18 Stunden zugegeben. Man rührt noch 3 Stunden bei 30°C, versetzt mit 10 ml Toluol, stellt mit Salzsäure (10%) auf pH 1-2 und rührt 30 min nach. Der Feststoff wird abgesaugt, zweimal abwechselnd mit Wasser und Heptan gewaschen und getrocknet. Man erhält die Titelverbindung als Feststoff in einer HPLC-Reinheit von 99 % Fläche (1,53g, 72% d. Th).
LC-MS: M+H = 319 (86%).
1H-NMR (400 MHz, DMSO-D₆): δ (ppm) = 11.3 (s, 1H), 7.59 (d,1H), 6.96 (dd, 1H), 6.93-7.00 (m, 1H), 4.50 (q, 4H), 1.37 (t, 6H).

### Beispiel 10:

### Herstellung von 2-[(4,6-Dimethoxy-1,3,5-triazin-2-yl)oxy]-3-phenyl-1H-indol (Durchführung analog zu Organic Letters (2010) 2306 Beispiele in Table 4 (Supplement Seite S-12 General Procedure):

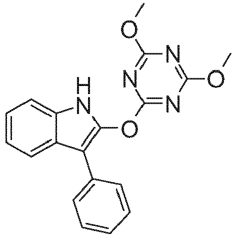

3-Phenyl-1,3-dihydro-2H-indol-2-on (0,25g; 1 eq.), 2-Chlor-4,6-dimethoxy-1,3,5-triazin (0,20g; 1 eq.) und Cäsiumcarbonat (0,37g, 1 eq.) werden vorgelegt und 20 ml N,N-Dimethylformamid (Wassergehalt < 0,1%) hinzugegeben. Der Ansatz wird 90 min bei 23°C gerührt. Eine HPLC-Analytik (Detektion bei 210 nm, Angaben in Flächenprozent, Lösungsmittelsignal wird nicht integriert) zeigt 3% 3-Phenyl-1,3-dihydro-2H-indol-2-on, 1,5% 2-Chlor-4,6-dimethoxy-1,3,5-triazin, 68% eines Hauptproduktes und kleine Nebenkomponenten. Der Ansatz wird auf 100 ml Wasser gegeben und mit verdünnter Salzsäure auf pH 4 gestellt. Der ausgefallene Feststoff wird abfiltriert und mit Wasser gewaschen. Man erhält eine Mischung, welche nach HPLC-Analytik (Detektion bei 210 nm, Angaben in Flächenprozent) zu 71% aus einem Hauptprodukt besteht (0,37g, 66% d. Th.). 100 mg der Mischung werden durch Säulenchromatographie (Eluent Essigsäureethylester und n-Heptan 1:1) gereinigt und die vereinigten Produktfraktionen im Vakuum eingeengt. Man erhält das Reaktionsprodukt als farblosen Feststoff in einer HPLC-Reinheit von 93% Fläche. Eine Strukturaufklärung per 2D-NMR belegt, dass es sich nicht um das in 3-Position arylierte Produkt (3-(4,6-Dimethoxy-1,3,5-triazin-2-yl)-3-phenyl-1,3-dihydro-2H-indol-2-on), sondern um das O-arylierte Produkt (2-[(4,6-Dimethoxy-1,3,5-triazin-2-yl)oxy]-3-phenyl-1H-indol) handelt.
LC-MS: M+H = 349 (96%).
1H-NMR (600 MHz, CDCl₃): δ (ppm) = 8.95 (s, breit, 1H), 7.82 (d, 1H), 7.65 (dd, 2H), 7.41 (t, 2H), 7.37 (d, 1H), 7.24 (q, 2H), 7.19 (t, 1H), 3.98 (s, 6H).

## Patentansprüche

1. Verfahren zur Herstellung von Verbindungen der Formel (3) 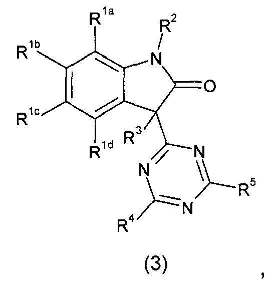 worin
R^{1a} bis R^{1d} unabhängig voneinander ausgewählt sind aus der Gruppe bestehend aus Wasserstoff, Fluor, Chlor, Brom, Iod sowie aus
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₈)-Cycloalkyl wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₁-C₆)-Alkylthio, wobei der Alkylthiorest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist, sowie aus
(C₃-C₇)-Cycloalkylthio, wobei der Cycloalkylthiorest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist, sowie aus Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R² für
Wasserstoff,
(C₁-C₆)-Alkyl, wobei der Alkylrest unsubstituiert oder substituiert ist durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist, oder
Benzyl, wobei das Benzyl unsubstituiert ist oder substituiert ist durch einen oder mehrere Substituenten, ausgewählt aus der Gruppe bestehend aus Fluor, Chlor, Brom, Iod, Nitro, (C₁-C₄)-Alkyl, (C₁-C₄)-Alkoxy oder (C₃-C₇)-Cycloalkyl oder (C₁-C₄)-Alkylthio sowie aus COOR^{a}, worin R^{a} für ein (C₁-C₄)-Alkyl steht, und -CONR^{b'}R^{b"} oder -CONHR^{b"}worin R^{b'} und R^{b"} jeweils unabhängig voneinander für ein (C₁-C₄)-Alkyl steht, wobei jeweils zwei Substituenten am N-Atom zusammen gegebenenfalls einen unsubstituierten oder substituierten Ring bilden,
steht,
R³ für Wasserstoff oder Methyl steht,
R⁴ und R⁵ unabhängig voneinander jeweils für
Wasserstoff,
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₁-C₆)-Alkoxy, wobei der Alkoxyrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist, stehen,
wobei ein Oxindol (1) 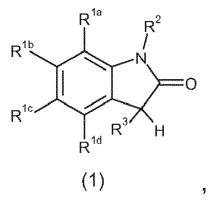 worin
R^{1a} bis R^{1d} sowie R² und R³ wie in Formel (3) definiert sind, in einem Lösungsmittel umgesetzt wird mit einem Triazin (2) 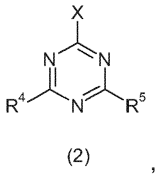 worin R⁴ und R⁵ wie in Formel (3) definiert sind, und
X als Abgangsgruppe für Cl, Br, I, Alkoxy, Alkylsulfonyl, (Alkylsulfonyl)oxy, Haloalkylsulfonyl, Phenylsulfonyl oder Toluol-4-Sulfonyl steht,
**dadurch gekennzeichnet, dass** die Umsetzung in Gegenwart von
- Kaliumcarbonat oder Natriumcarbonat,
- Lithiumhydroxid, Natriumhydroxid, Kaliumhydroxid, Bariumhydroxid oder Tertalkylammoniumhydroxid,
- Kaliumphosphat (K₃PO₄), Kaliumhydrogenphosphate (K₂HPO₄) oder Natriumphosphat, oder
- in einer mindestens zwei der vorgenannten Basen umfassenden Mischung erfolgt.

2. Verfahren zur Herstellung von Verbindungen der Formel (3) nach Anspruch ₁, **dadurch gekennzeichnet, dass** R^{1a} bis R^{1d} unabhängig voneinander ausgewählt sind aus der Gruppe bestehend aus Wasserstoff, Fluor, Chlor, Brom, Iod sowie aus
(C₁-C₆)-Alkyl, wobei der Alkylrest unsubstituiert ist oder durch einen oder mehrere Substituenten, ausgewählt aus der Gruppe bestehend aus Fluor und Chlor substituiert ist, und
(C₃-C₇)-Cycloalkyl, wobei der Cycloalkylrest unsubstituiert ist oder durch einen oder mehrere Substituenten, ausgewählt aus der aus Fluor und Chlor, (C₁-C₄)-Alkyl oder (C₃-C₇)-Cycloalkyl oder (C₁-C₄)-Alkoxy bestehenden Gruppe, substituiert ist.

3. Verfahren zur Herstellung von Verbindungen der Formel (3) nach Anspruch ₂, **dadurch gekennzeichnet, dass** R^{1a} bis R^{1d} unabhängig voneinander ausgewählt sind aus der Gruppe bestehend aus Fluor, Chlor, Trifluormethyl, Trifluormethoxy und Methoxy.

4. Verfahren zur Herstellung von Verbindungen der Formel (3) nach Anspruch 3, **dadurch gekennzeichnet, dass** R^{1a} für Fluor oder Chlor steht.

5. Verfahren zur Herstellung von Verbindungen der Formel (3) nach einem der vorhergehenden Ansprüche, **dadurch gekennzeichnet, dass** R² für
- Wasserstoff, oder für jeweils
- unsubstituiertes Methyl, Ethyl und Benzyl
steht.

6. Verfahren zur Herstellung von Verbindungen der Formel (3) nach Anspruch 5, **dadurch gekennzeichnet, dass** R² für Wasserstoff steht.

7. Verfahren zur Herstellung von Verbindungen der Formel (3) nach einem der vorhergehenden Ansprüche, **dadurch gekennzeichnet, dass** R⁴ und R⁵ unabhängig voneinander für
- unsubstituiertes (C₁-C₄)-Alkyl, und
- unsubstituiertes (C₁-C₄)-Alkoxy
stehen.

8. Verfahren zur Herstellung von Verbindungen der Formel (3) nach einem der vorhergehenden Ansprüche, **dadurch gekennzeichnet, dass** X für Chlor steht.

9. Verfahren zur Herstellung von Verbindungen der Formel (3) nach einem der vorhergehenden Ansprüche, **dadurch gekennzeichnet, dass** die Umsetzung der Edukte in
- einem polaren, oder
- einem unpolaren Lösungsmittel, oder
- einem Gemisch aus einem polaren oder unpolaren Lösungsmittel durchgeführt wird.

10. Verfahren zur Herstellung von Verbindungen der Formel (3) nach einem der vorhergehenden Ansprüche, **dadurch gekennzeichnet, dass** die Umsetzung wasserfrei in Gegenwart der in Anspruch 1 genannten Basen erfolgt.

11. Verfahren zur Herstellung von Verbindungen der Formel (3) nach einem der vorhergehenden Ansprüche, **dadurch gekennzeichnet, dass** zu einem Äquivalent des Oxindols (1) 1.1 bis 1.4 Äquivalente des Triazins (2) gegeben werden.

12. Verfahren zur Herstellung von Verbindungen der Formel (3) nach einem der vorhergehenden Ansprüche, **dadurch gekennzeichnet, dass** die Zugabe der Edukte in einer Portion oder gleichmäßig dosiert über einen Zeitraum von bis zu 24 Stunden erfolgt.

13. Verfahren zur Herstellung von Verbindungen der Formel (3) nach einem der vorhergehenden Ansprüche, **dadurch gekennzeichnet, dass** die Umsetzung der Edukte bei einer Temperatur in dem Bereich von - 20° bis 150° C durchgeführt wird.

14. Verbindungen der Formel (3) 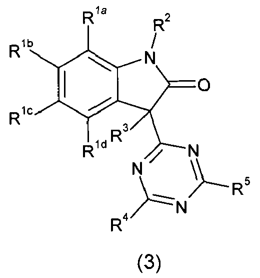 und deren Salze (3") 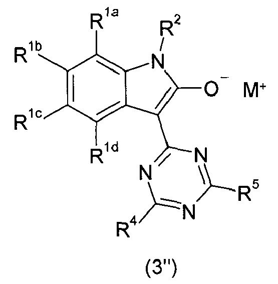 worin
R^{1a} bis R^{1d} unabhängig voneinander ausgewählt sind aus der Gruppe bestehend aus Wasserstoff, Fluor, Chlor, Brom, Iod sowie aus
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₈)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₁-C₆)-Alkylthio, wobei der Alkylthiorest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
(C₃-C₇)-Cycloalkylthio, wobei der Cycloalkylthiorest unsubstituiert ist oder durch einen oder mehrere Substituenten, ausgewählt aus der Fluor, Chlor, (C₁-C₄)-Alkyl oder (C₁-C₄)-Alkoxy bestehenden Gruppe, substituiert ist, sowie aus
Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R² für
Wasserstoff,
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist, oder
Benzyl, wobei das Benzyl unsubstituiert ist oder substituiert ist durch einen oder mehrere Substituenten, ausgewählt aus der Gruppe bestehend aus Fluor, Chlor, Brom, Iod, Nitro, (C₁-C₄)-Alkyl, (C₁-C₄)-Alkoxy oder (C₃-C₇)-Cycloalkyl oder (C₁-C₄)-Alkylthio sowie aus COOR^{a}, worin R^{a} für ein (C₁-C₄)-Alkyl steht, und -CONR^{b'}R^{b"} oder -CONHR^{b"} worin R^{b'} und R^{b"} jeweils unabhängig voneinander für ein (C₁-C₄)-Alkyl steht, wobei jeweils zwei Substituenten am N-Atom zusammen gegebenenfalls einen unsubstituierten oder substituierten Ring bilden,
steht,
R³ für
Wasserstoff oder Methyl
steht,
R⁴ und R⁵ unabhängig voneinander jeweils für
Wasserstoff,
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₁-C₆)-Alkoxy, wobei der Alkoxyrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist, stehen,
wobei in den Salzen der allgemeinen Formel (3")
M für Li, Na, K, N(R^{c})₄, mit R^{c} = H oder C₁-C₆ Alkyl, Cs, Ba, Mg, Ca und Zn steht und die Anzahl der Gegenionen M⁺ sich nach der jeweiligen Ladung richtet, so dass eine insgesamt neutrale Verbindung der allgemeinen Formel (3") entsteht.

15. Verwendung einer Verbindung der Formel (3) oder deren Salze (3") nach Anspruch 14 zur Herstellung von Pflanzenschutzmitteln.

16. Verwendung einer Verbindung der Formel (3) oder deren Salze nach Anspruch 14 zur Herstellung von Herbiziden oder von Intermediaten zur Herstellung von Herbiziden.

17. Verwendung einer Verbindung der Formel (3) oder deren Salze nach Anspruch 14 zur Herstellung von N-[2-(1,3,5-Triazin-2-ylcarbonyl)phenyl]alkylsulfonamiden.

18. Verwendung einer Verbindung der Formel (3) oder deren Salze nach Anspruch 14 zur Herstellung von N-Alkyl-N-[2-(1,3,5-triazin-2-ylcarbonyl)phenyl] alkylsulfonamiden.

## Claims

1. A process for the preparation of compounds of formula (3) 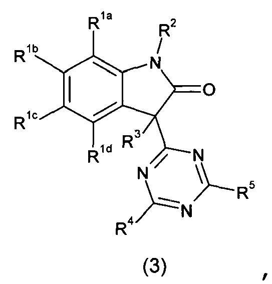 in which
R^{1a} to R^{1d}, independently of one another, are selected from the group consisting of hydrogen, fluorine, chlorine, bromine, iodine, and also from
(C₁-C₆)-alkyl, where the alkyl radical is unsubstituted or is substituted by one or more substituents selected from the group consisting of fluorine, chlorine, (C₁-C₄) -alkoxy or (C₃-C₇)-cycloalkyl,
(C₃-C₈)-cycloalkyl, where the cycloalkyl radical is unsubstituted or is substituted by one or more substituents selected from the group consisting of fluorine, chlorine, (C₁-C₄)-alkyl or (C₃-C₇)-cycloalkyl or (C₁-C₄)-alkoxy,
(C₁-C₆)-alkoxy, where the alkoxy radical is unsubstituted or is substituted by one or more substituents selected from the group consisting of fluorine, chlorine, (C₁-C₄)-alkoxy or (C₃-C₇)-cycloalkyl,
(C₃-C₇)-cycloalkoxy, where the cycloalkoxy radical is unsubstituted or is substituted by one or more substituents selected from the group consisting of fluorine, chlorine, (C₁-C₄)-alkyl or (C₁-C₄)-alkoxy,
(C₁-C₆)-alkylthio, where the alkylthio radical is unsubstituted or is substituted by one or more substituents selected from the group consisting of fluorine, chlorine, (C₁-C₄)-alkyl or (C₁-C₉)-alkoxy, and also from
(C₃-C₇)-cycloalkylthio, where the cycloalkylthio radical is unsubstituted or is substituted by one or more substituents selected from the group consisting of fluorine, chlorine, (C₁-C₄)-alkyl or (C₁-C₄)-alkoxy, and also from
phenyl or 1-naphthyl or 2-naphthyl or a five- or six-membered heteroaromatic ring having 1 to 2 heteroatoms, where the heteroatoms, independently of one another, are selected from the group consisting of O or N and where the aryl or heteroaryl radical is unsubstituted or is substituted by one or more substituents selected from the group consisting of fluorine, chlorine, bromine, iodine, (C₁-C₄)-alkyl, (C₁-C₄) -alkoxy or (C₃-C₇) -cycloalkyl or (C₁-C₄)-alkylthio, and
R² is
hydrogen,
(C₁-C₆)-alkyl, where the alkyl radical is unsubstituted or is substituted by one or more substituents selected from the group consisting of fluorine, chlorine, (C₁-C₄)-alkoxy or (C₃-C₇)-cycloalkyl, or
benzyl, where the benzyl is unsubstituted or is substituted by one or more substituents selected from the group consisting of fluorine, chlorine, bromine, iodine, nitro, (C₁-C₄) -alkyl, (C₁-C₄) - alkoxy or (C₃-C₇)-cycloalkyl or (C₁-C₄)-alkylthio and also from COOR^{a}, in which R^{a} is a (C₁-C₄)-alkyl, and -CONR^{b'}R^{b"} or -CONHR^{b"}, in which R^{b'} and R^{b"} are each independently of one another a (C₁-C₄)-alkyl, where in each case two substituents on the N atom together optionally form an unsubstituted or substituted ring,
R³ is hydrogen or methyl,
R⁴ and R⁵, independently of one another, are in each case
hydrogen,
(C₁-C₆)-alkyl, where the alkyl radical is unsubstituted or is substituted by one or more substituents selected from the group consisting of fluorine, chlorine, (C₁-C₄)-alkoxy or (C₃-C₇)-cycloalkyl,
(C₁-C₆)-alkoxy, where the alkoxy radical is unsubstituted or is substituted by one or more substituents selected from the group consisting of fluorine, chlorine, (C₁-C₄)-alkoxy or (C₃-C₇)-cycloalkyl,
where an oxindole (1) 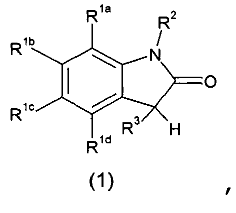 in which
R^{1a} to R^{1d} and R² and R³ are as defined in formula (3), is reacted in a solvent with a triazine (2) 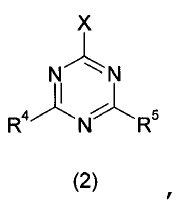 in which
R⁴ and R⁵ are as defined in formula (3), and
X, as leaving group, is Cl, Br, I, alkoxy, alkylsulfonyl, (alkylsulfonyl)oxy, haloalkylsulfonyl, phenylsulfonyl or toluene-4-sulfonyl,
which comprises carrying out the reaction in the presence of
- potassium carbonate or sodium carbonate,
- lithium hydroxide, sodium hydroxide, potassium hydroxide, barium hydroxide or tert-alkylammonium hydroxide,
- potassium phosphate (K₃PO₄), potassium hydrogenphosphate (K₂HPO₄) or sodium phosphate or
- in a mixture comprising at least two of the aforementioned bases.

2. The process for the preparation of compounds of formula (3) as claimed in claim 1, wherein R^{1a} to R^{1d}, independently of one another, are selected from the group consisting of hydrogen, fluorine, chlorine, bromine, iodine, and also from
(C₁-C₆)-alkyl, where the alkyl radical is unsubstituted or is substituted by one or more substituents selected from the group consisting of fluorine and chlorine, and
(C₃-C₇)-cycloalkyl, where the cycloalkyl radical is unsubstituted or is substituted by one or more substituents selected from the group consisting of fluorine and chlorine, (C₁-C₄) -alkyl or (C₃-C₇)-cycloalkyl or (C₁-C₄)-alkoxy.

3. The process for the preparation of compounds of formula (3) as claimed in claim 2, wherein R^{1a} to R^{1d}, independently of one another, are selected from the group consisting of fluorine, chlorine, trifluoromethyl, trifluoromethoxy and methoxy.

4. The process for the preparation of compounds of formula (3) as claimed in claim 3, wherein R^{1a} is fluorine or chlorine.

5. The process for the preparation of compounds of formula (3) as claimed in one of the preceding claims,
wherein
R² is
- hydrogen, or is in each case
- unsubstituted methyl, ethyl and benzyl.

6. The process for the preparation of compounds of formula (3) as claimed in claim 5, wherein R² is hydrogen.

7. The process for the preparation of compounds of formula (3) as claimed in one of the preceding claims, wherein R⁴ and R⁵, independently of one another, are
- unsubstituted (C₁-C₄)-alkyl, and
- unsubstituted (C₁-C₄)-alkoxy.

8. The process for the preparation of compounds of formula (3) as claimed in one of the preceding claims, wherein X is chlorine.

9. The process for the preparation of compounds of formula (3) as claimed in one of the preceding claims, wherein the reaction of the starting materials is carried out in
- a polar or
- a nonpolar solvent, or
- a mixture of a polar or nonpolar solvent.

10. The process for the preparation of compounds of formula (3) as claimed in one of the preceding claims, wherein the reaction takes place under anhydrous conditions in the presence of the bases mentioned in claim 1.

11. The process for the preparation of compounds of formula (3) as claimed in one of the preceding claims, wherein 1.1 to 1.4 equivalents of the triazine (2) are added to one equivalent of the oxindole (1).

12. The process for the preparation of compounds of formula (3) as claimed in one of the preceding claims, wherein the addition of the starting materials takes place in one portion or equally dosed over a period of up to 24 hours.

13. The process for the preparation of compounds of formula (3) as claimed in one of the preceding claims, wherein the reaction of the starting materials is carried out at a temperature in the range from -20° to 150°C.

14. A compound of formula (3) 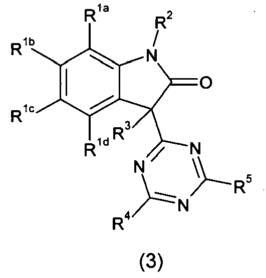 and salts thereof (3") 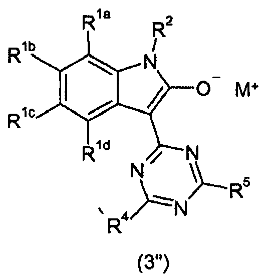 in which
R^{1a} to R^{1d}, independently of one another, are selected from the group consisting of hydrogen, fluorine, chlorine, bromine, iodine, and also from
(C₁-C₆)-alkyl, where the alkyl radical is unsubstituted or is substituted by one or more substituents selected from the group consisting of fluorine, chlorine, (C₁-C₄)-alkoxy or (C₃-C₇)-cycloalkyl,
(C₃-C₈)-cycloalkyl, where the cycloalkyl radical is unsubstituted or is substituted by one or more substituents selected from the group consisting of fluorine, chlorine, (C₁-C₄) -alkyl or (C₃-C₇)-cycloalkyl or (C₁-C₄)-alkoxy,
(C₁-C₆)-alkoxy, where the alkoxy radical is unsubstituted or is substituted by one or more substituents selected from the group consisting of fluorine, chlorine, (C₁-C₄)-alkoxy or (C₃-C₇)-cycloalkyl,
(C₃-C₇)-cycloalkoxy, where the cycloalkoxy radical is unsubstituted or is substituted by one or more substituents selected from the group consisting of fluorine, chlorine, (C₁-C₄)-alkyl or (C₁-C₄)-alkoxy,
(C₁-C₆)-alkylthio, where the alkylthio radical is unsubstituted or is substituted by one or more substituents selected from the group consisting of fluorine, chlorine, (C₁-C₄)-alkyl or (C₁-C₄)-alkoxy,
(C₃-C₇)-cycloalkylthio, where the cycloalkylthio radical is unsubstituted or is substituted by one or more substituents selected from the group consisting of fluorine, chlorine, (C₁-C₄)-alkyl or (C₁-C₄)-alkoxy, and also from
phenyl or 1-naphthyl or 2-naphthyl or a five- or six-membered heteroaromatic ring having 1 to 2 heteroatoms, where the heteroatoms, independently of one another, are selected from the group consisting of O or N and where the aryl or heteroaryl radical is unsubstituted or is substituted by one or more substituents selected from the group consisting of fluorine, chlorine, bromine, iodine, (C₁-C₄) -alkyl, (C₁-C₄) -alkoxy or (C₃-C₇)-cycloalkyl or (C₁-C₄)-alkylthio, and
R² is
hydrogen,
(C₁-C₆)-alkyl, where the alkyl radical is unsubstituted or is substituted by one or more substituents selected from the group consisting of fluorine, chlorine, (C₁-C₄)-alkoxy or (C₃-C₇)-cycloalkyl, or
benzyl, where the benzyl is unsubstituted or is substituted by one or more substituents selected from the group consisting of fluorine, chlorine, bromine, iodine, nitro, (C₁-C₄)-alkyl, (C₁-C₄)-alkoxy or (C₃-C₇)-cycloalkyl or (C₁-C₄)-alkylthio and also from COOR^{a}, in which R^{a} is a (C₁-C₄)-alkyl, and -CONR^{b'}R^{b"} or -CONHR^{b"}, in which R^{b'} and R^{b"} are each independently of one another a (C₁-C₄)-alkyl, where in each case two substituents on the N atom together optionally form an unsubstituted or substituted ring,
R3 is
Hydrogen or methyl,
R⁴ and R⁵, independently of one another, are in each case
hydrogen,
(C₁-C₆)-alkyl, where the alkyl radical is unsubstituted or is substituted by one or more substituents selected from the group consisting of fluorine, chlorine, (C₁-C₄)-alkoxy or (C₃-C₇)-cycloalkyl,
(C₁-C₆)-alkoxy, where the alkoxy radical is unsubstituted or is substituted by one or more substituents selected from the group consisting of fluorine, chlorine, (C₁-C₄)-alkoxy or (C₃-C₇)-cycloalkyl,
where, in the salts of formula (3''),
M is Li, Na, K, N(R^{c})₄, where R^{c} = H or C₁₋C₆ alkyl, Cs, Ba, Mg, Ca and Zn, and the number of counter ions M⁺ is governed by the particular charge such that an overall neutral compound of formula (3") is formed.

15. The use of a compound of formula (3) or salts thereof (3") as claimed in claim 14 for producing crop protection agents.

16. The use of a compound of formula (3) or salts thereof as claimed in claim 14 for producing herbicides or intermediates for producing herbicides.

17. The use of a compound of formula (3) or salts thereof as claimed in claim 14 for producing N-[2-(1,3,5-triazin-2-ylcarbonyl)phenyl]alkylsulfonamides.

18. The use of a compound of formula (3) or salts thereof as claimed in claim 14 for producing N-alkyl-N-[2-(1,3,5-triazin-2-ylcarbonyl)-phenyl]alkylsulfonamides.

## Revendications

1. Procédé de fabrication de composés de formule (3) 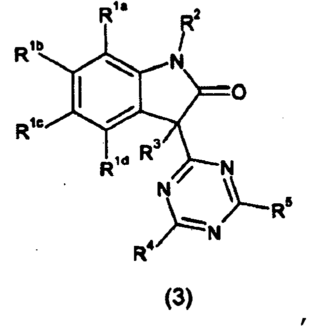 dans laquelle
R^{1a} à R^{1d} sont choisis indépendamment les uns des autres dans le groupe constitué par hydrogène, fluor, chlore, brome, iode, ainsi que par
alkyle en (C₁-C₆), le radical alkyle étant non substitué ou substitué par un ou plusieurs substituants, choisis dans le groupe constitué par fluor, chlore, alcoxy en (C₁-C₄) ou cycloalkyle en (C₃-C₇),
cycloalkyle en (C₃-C₈), le radical cycloalkyle étant non substitué ou substitué par un ou plusieurs substituants, choisis dans le groupe constitué par fluor, chlore, alkyle en (C₁-C₄) ou cycloalkyle en (C₃-C₇) ou alcoxy en (C₁-C₄),
alcoxy en (C₁-C₆), le radical alcoxy étant non substitué ou substitué par un ou plusieurs substituants, choisis dans le groupe constitué par fluor, chlore, alcoxy en (C₁-C₄) ou cycloalkyle en (C₃-C₇),
cycloalcoxy en (C₃-C₇), le radical cycloalcoxy étant non substitué ou substitué par un ou plusieurs substituants, choisis dans le groupe constitué par fluor, chlore, alkyle en (C₁-C₄) ou alcoxy en (C₁-C₄), alkylthio en (C₁-C₆), le radical alkylthio étant non substitué ou substitué par un ou plusieurs substituants, choisis dans le groupe constitué par fluor, chlore, alkyle en (C₁-C₄) ou alcoxy en (C₁-C₄), ainsi que par
cycloalkylthio en (C₃-C₇), le radical cycloalkylthio étant non substitué ou substitué par un ou plusieurs substituants, choisis dans le groupe constitué par fluor, chlore, alkyle en (C₁-C₄) ou alcoxy en (C₁-C₄), ainsi que par
phényle ou 1-naphtyle ou 2-naphtyle ou un cycle hétéroaromatique à cinq ou six éléments contenant 1 à 2 hétéroatomes, les hétéroatomes étant choisis indépendamment les uns des autres dans le groupe constitué par O ou N, et le radical aryle ou hétéroaryle étant non substitué ou substitué par un ou plusieurs substituants, choisis dans le groupe constitué par fluor, chlore, brome, iode, alkyle en (C₁-C₄), alcoxy en (C₁-C₄) ou cycloalkyle en (C₃-C₇) ou alkylthio en (C₁-C₄), et
R² représente
hydrogène,
alkyle en (C₁-C₆), le radical alkyle étant non substitué ou substitué par un ou plusieurs substituants, choisis dans le groupe constitué par fluor, chlore, alcoxy en (C₁-C₄) ou cycloalkyle en (C₃-C₇), ou
benzyle, le benzyle étant non substitué ou substitué par un ou plusieurs substituants, choisis dans le groupe constitué par fluor, chlore, brome, iode, nitro, alkyle en (C₁-C₄), alcoxy en (C₁-C₄) ou cycloalkyle en (C₃-C₇) ou alkylthio en (C₁-C₄), ainsi que par COOR^{a}, R^{a} représentant un alkyle en (C₁-C₄), et -CONR^{b'}R^{b"} ou - CONHR^{b"} , R^{b'} et R^{b"} représentant chacun indépendamment l'un de l'autre alkyle en (C₁-C₄), deux substituants sur l'atome N formant à chaque fois éventuellement ensemble un cycle non substitué ou substitué,
R³ représente hydrogène ou méthyle,
R⁴ et R⁵ représentent chacun indépendamment l'un de l'autre
hydrogène,
alkyle en (C₁-C₆), le radical alkyle étant non substitué ou substitué par un ou plusieurs substituants, choisis dans le groupe constitué par fluor, chlore, alcoxy en (C₁-C₄) ou cycloalkyle en (C₃-C₇),
alcoxy en (C₁-C₆), le radical alcoxy étant non substitué ou substitué par un ou plusieurs substituants, choisis dans le groupe constitué par fluor, chlore, alcoxy en (C₁-C₄) ou cycloalkyle en (C₃-C₇),
selon lequel un oxindole (1) 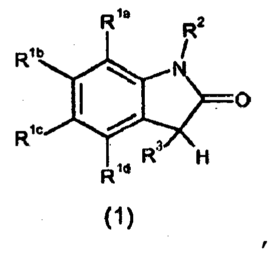 dans lequel
R^{1a} à R^{1d}, ainsi que R² et R³, sont tels que définis dans la formule (3), est mis en réaction dans un solvant avec une triazine (2) 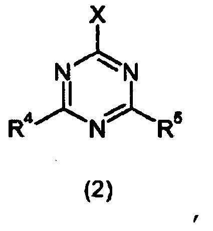 dans laquelle
R⁴ et R⁵ sont tels que définis dans la formule (3), et
X représente en tant que groupe partant Cl, Br, I, alcoxy, alkylsulfonyle, (alkylsulfonyl)oxy, haloalkylsulfonyle, phénylsulfonyle ou toluène-4-sulfonyle,
**caractérisé en ce que** la réaction a lieu en présence de
- carbonate de potassium ou carbonate de sodium,
- hydroxyde de lithium, hydroxyde de sodium, hydroxyde de potassium, hydroxyde de baryum ou hydroxyde de tétraalkylammonium,
- phosphate de potassium (K₃PO₄), hydrogénophosphate de potassium (K₂HPO₄) ou phosphate de sodium, ou
- dans un mélange comprenant au moins deux des bases susmentionnées.

2. Procédé de fabrication de composés de formule (3) selon la revendication 1, **caractérisé en ce que** R^{1a} à R^{1d} sont choisis indépendamment les uns des autres dans le groupe constitué par hydrogène, fluor, chlore, brome, iode, ainsi que par alkyle en (C₁-C₆), le radical alkyle étant non substitué ou substitué par un ou plusieurs substituants, choisis dans le groupe constitué par fluor et chlore, et
cycloalkyle en (C₃-C₇), le radical cycloalkyle étant non substitué ou substitué par un ou plusieurs substituants, choisis dans le groupe constitué par fluor et chlore, alkyle en (C₁-C₄) ou cycloalkyle en (C₃-C₇) ou alcoxy en (C₁-C₄).

3. Procédé de fabrication de composés de formule (3) selon la revendication 2, **caractérisé en ce que** R^{1a} à R^{1d} sont choisis indépendamment les uns des autres dans le groupe constitué par fluor, chlore, trifluorométhyle, trifluorométhoxy et méthoxy.

4. Procédé de fabrication de composés de formule (3) selon la revendication 3, **caractérisé en ce que** R^{1a} représente fluor ou chlore.

5. Procédé de fabrication de composés de formule (3) selon l'une quelconque des revendications précédentes, **caractérisé en ce que**
R² représente
- hydrogène ou
- méthyle, éthyle et benzyle, chacun non substitués.

6. Procédé de fabrication de composés de formule (3) selon la revendication 5, **caractérisé en ce que** R² représente hydrogène.

7. Procédé de fabrication de composés de formule (3) selon l'une quelconque des revendications précédentes, **caractérisé en ce que** R⁴ et R⁵ représentent indépendamment l'un de l'autre
- alkyle en (C₁-C₄) non substitué et
- alcoxy en (C₁-C₄) non substitué.

8. Procédé de fabrication de composés de formule (3) selon l'une quelconque des revendications précédentes, **caractérisé en ce que** X représente chlore.

9. Procédé de fabrication de composés de formule (3) selon l'une quelconque des revendications précédentes, **caractérisé en ce que** la réaction des réactifs est réalisée dans
- un solvant polaire ou
- apolaire ou
- un mélange d'un solvant polaire ou apolaire.

10. Procédé de fabrication de composés de formule (3) selon l'une quelconque des revendications précédentes, **caractérisé en ce que** la réaction a lieu sous forme anhydre en présence des bases indiquées dans la revendication 1.

11. Procédé de fabrication de composés de formule (3) selon l'une quelconque des revendications précédentes, **caractérisé en ce que** 1,1 à 1,4 équivalent de triazine (2) est ajouté à un équivalent de l'oxindole (1).

12. Procédé de fabrication de composés de formule (3) selon l'une quelconque des revendications précédentes, **caractérisé en ce que** l'ajout des réactifs a lieu en une portion ou dosé de manière uniforme pendant une période de jusqu'à 24 heures.

13. Procédé de fabrication de composés de formule (3) selon l'une quelconque des revendications précédentes, **caractérisé en ce que** la réaction des réactifs est réalisée à une température dans la plage allant de -20 à 150 °C.

14. Composés de formule (3) 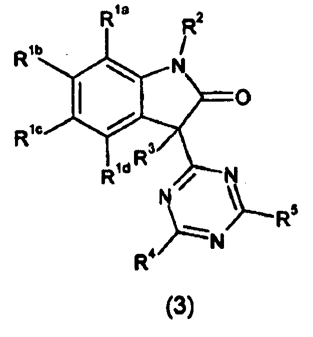 et leurs sels (3") 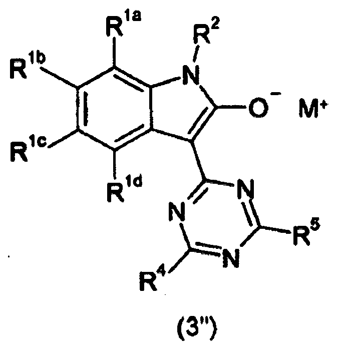 dans lesquels
R^{1a} à R^{1d} sont choisis indépendamment les uns des autres dans le groupe constitué par hydrogène, fluor, chlore, brome, iode, ainsi que par
alkyle en (C₁-C₆), le radical alkyle étant non substitué ou substitué par un ou plusieurs substituants, choisis dans le groupe constitué par fluor, chlore, alcoxy en (C₁-C₄) ou cycloalkyle en (C₃-C₇),
cycloalkyle en (C₃-C₈), le radical cycloalkyle étant non substitué ou substitué par un ou plusieurs substituants, choisis dans le groupe constitué par fluor, chlore, alkyle en (C₁-C₄) ou cycloalkyle en (C₃-C₇) ou alcoxy en (C₁-C₄),
alcoxy en (C₁-C₆), le radical alcoxy étant non substitué ou substitué par un ou plusieurs substituants, choisis dans le groupe constitué par fluor, chlore, alcoxy en (C₁-C₄) ou cycloalkyle en (C₃-C₇),
cycloalcoxy en (C₃-C₇), le radical cycloalcoxy étant non substitué ou substitué par un ou plusieurs substituants, choisis dans le groupe constitué par fluor, chlore, alkyle en (C₁-C₄) ou alcoxy en (C₁-C₄), alkylthio en (C₁-C₆), le radical alkylthio étant non substitué ou substitué par un ou plusieurs substituants, choisis dans le groupe constitué par fluor, chlore, alkyle en (C₁-C₄) ou alcoxy en (C₁-C₄), cycloalkylthio en (C₃-C₇), le radical cycloalkylthio étant non substitué ou substitué par un ou plusieurs substituants, choisis dans le groupe constitué par fluor, chlore, alkyle en (C₁-C₄) ou alcoxy en (C₁-C₄), ainsi que par
phényle ou 1-naphtyle ou 2-naphtyle ou un cycle hétéroaromatique à cinq ou six éléments contenant 1 à 2 hétéroatomes, les hétéroatomes étant choisis indépendamment les uns des autres dans le groupe constitué par O ou N, et le radical aryle ou hétéroaryle étant non substitué ou substitué par un ou plusieurs substituants, choisis dans le groupe constitué par fluor, chlore, brome, iode, alkyle en (C₁-C₄), alcoxy en (C₁-C₄) ou cycloalkyle en (C₃-C₇) ou alkylthio en (C₁-C₄), et
R² représente
hydrogène,
alkyle en (C₁-C₆), le radical alkyle étant non substitué ou substitué par un ou plusieurs substituants, choisis dans le groupe constitué par fluor, chlore, alcoxy en (C₁-C₄) ou cycloalkyle en (C₃-C₇), ou
benzyle, le benzyle étant non substitué ou substitué par un ou plusieurs substituants, choisis dans le groupe constitué par fluor, chlore, brome, iode, nitro, alkyle en (C₁-C₄), alcoxy en (C₁-C₄) ou cycloalkyle en (C₃-C₇) ou alkylthio en (C₁-C₄), ainsi que par COOR^{a}, R^{a} représentant un alkyle en (C₁-C₄), et -CONR^{b'}R^{b"} ou - CONHR^{b"}, R^{b'} et R^{b"} représentant chacun indépendamment l'un de l'autre alkyle en (C₁-C₄), deux substituants sur l'atome N formant à chaque fois éventuellement ensemble un cycle non substitué ou substitué,
R³ représente hydrogène ou méthyle,
R⁴ et R⁵ représentent chacun indépendamment l'un de l'autre
hydrogène,
alkyle en (C₁-C₆), le radical alkyle étant non substitué ou substitué par un ou plusieurs substituants, choisis dans le groupe constitué par fluor, chlore, alcoxy en (C₁-C₄) ou cycloalkyle en (C₃-C₇),
alcoxy en (C₁-C₆), le radical alcoxy étant non substitué ou substitué par un ou plusieurs substituants, choisis dans le groupe constitué par fluor, chlore, alcoxy en (C₁-C₄) ou cycloalkyle en (C₃-C₇),
dans les sels de formule générale (3''),
M représentant Li, Na, K, N(R^{c})₄, avec R^{c} = H ou alkyle en C₁-C₆, Cs, Ba, Mg, Ca et Zn, et le nombre de contre-ions M⁺ étant déterminé par la charge respective de manière à former un composé de formule générale (3'') neutre au total.

15. Utilisation d'un composé de formule (3) ou ses sels (3'') selon la revendication 14 pour la fabrication d'agents phytosanitaires.

16. Utilisation d'un composé de formule (3) ou ses sels selon la revendication 14 pour la fabrication d'herbicides ou d'intermédiaires pour la fabrication d'herbicides.

17. Utilisation d'un composé de formule (3) ou ses sels selon la revendication 14 pour la fabrication de N-[2-(1,3,5-triazin-2-ylcarbonyl)phényl]alkylsulfonamides.

18. Utilisation d'un composé de formule (3) ou ses sels selon la revendication 14 pour la fabrication de N-alkyl-N-[2-(1,3,5-triazin-2-ylcarbonyl)phényl]alkylsulfonamides.
